# EUROPEAN PATENT SPECIFICATION

(11) **EP 4 623 103 B1**
(45) Date of publication and mention of the grant of the patent: **04.02.2026**
(21) Application number: 24700542.4
(22) Date of filing: 05.01.2024
(51) Int. Cl.: C12Q 1/6855, C12Q 1/6806

(54) **METHODS FOR OBTAINING CORRECTLY ASSEMBLED NUCLEIC ACIDS**
VERFAHREN ZUR KORREKTEN GEWINNUNG VON NUKLEINSÄUREN
PROCÉDÉS D'OBTENTION D'ACIDES NUCLÉIQUES CORRECTEMENT ASSEMBLÉS

(30) Priority: 06.01.2023 EP 23315001
(43) Date of publication of application: 01.10.2025
(73) Proprietor: DNA Script, 94270 Le Kremlin-Bicêtre (FR)
(72) Inventor: JEDDELOH, Jeffrey, 94270 LE KREMLIN-BICÊTRE (FR)
(74) Representative: Plasseraud IP
(86) International application number: PCT/EP2024/050196
(87) International publication number: WO 2024/146937

(56) References cited:
- WO-A1-2018/108328
- WO-A1-2022/056418
- WO-A1-2022/221853
- DELLEY CYRILLE L ET AL: "Modular barcode beads for microfluidic single cell genomics", SCIENTIFIC REPORTS, 25 May 2021 (2021-05-25), London, pages 10857 - 10857, XP055854041, Retrieved from the Internet <URL:https://www.nature.com/articles/s41598-021-90255-x.pdf> [retrieved on 20211022], DOI: 10.1038/s41598-021-90255-x
- LINDENBURG LAURENS ET AL: "Split & mix assembly of DNA libraries for ultrahigh throughput on-bead screening of functional proteins", vol. 48, no. 11, 19 June 2020 (2020-06-19), GB, pages e63 - e63, XP055927468, ISSN: 0305-1048, Retrieved from the Internet <URL:https://www.ncbi.nlm.nih.gov/pmc/articles/PMC7293038/pdf/gkaa270.pdf> DOI: 10.1093/nar/gkaa270

## Description

### FIELD

In some aspects, the invention relates to assembling perfect-sequence nucleic acids from nucleic acid segments.

### INTRODUCTION

Obtaining sequence-perfect copies of nucleic acids is a key problem in synthetic biology. Typically, the process of synthesizing a nucleic acid sequence of interest begins by making synthetic oligonucleotides that are designed to be assembled into the desired sequence of interest. Then, a series of lab procedures are performed to combine the oligonucleotides into a full-length nucleic acid. Errors occur in this process, such that the likelihood of obtaining perfect copies of the nucleic acid of interest is inversely proportional to the length of the desired nucleic acid sequence. Therefore, perfect copies of long sequences are often produced as a small portion of the total population of assembled nucleic acids.

The current state of the art uses colony-picking and sequencing of individual clones from a large population of clones. For example, assembled nucleic acids are inserted into plasmids that are expressed in a host such as *Escherichia coli (E. coli).* The host cells are plated on suitable growth media to produce separate colonies. Colony-specific plasmids are then sequenced individually to find plasmids that contain the desired full length, perfect-sequence inserts. Longer assembled sequences require more colony screening than shorter assembled sequences. The process of colony-picking, plasmid purification, and sequencing each clone individually is labor-intensive and time-consuming.

International patent application WO2022/221853 describes in vitro methods for retrieving assembled DNA molecules, using dilution steps and molecular barcodes.

Accordingly, there is a need for new ways to obtain perfect copies of nucleic acid sequences of interest.

### SUMMARY OF THE INVENTION

Methods are provided for cell-free identification and amplification of one or more correctly assembled nucleic acid comprising a sequence of interest from a mixture of nucleic acids. In particular, the subject methods use split-pool enzymatic DNA synthesis (EDS) to add a bead-specific index sequence to bead-bound clonally amplified assembled nucleic acids. High-throughput sequencing is used to identify one or more correctly assembled nucleic acids comprising a sequence of interest, wherein each bead-specific index sequence that is associated with a correctly assembled nucleic acid allows selective amplification of the correctly assembled nucleic acid using a primer having a sequence complementary to the bead-specific index sequence.

In some embodiments, a cell-free method of identifying and amplifying a correctly assembled nucleic acid comprising a sequence of interest is provided. The method comprises: ligating adapters to the 5' and 3' ends of a plurality of assembled nucleic acids, wherein the adapters each comprise an adapter primer binding site and an adapter capture sequence; binding the plurality of assembled nucleic acids to a plurality of beads, wherein each bead comprises a capture oligonucleotide that specifically hybridizes to the adapter capture sequence, preferably wherein no more than one assembled nucleic acid is bound to each bead; performing clonal amplification of the plurality of assembled nucleic acids that are bound to the beads using a first primer that hybridizes to the adapter capture sequence and a second primer that hybridizes to the adapter primer binding site, wherein amplicons produced by clonal amplification of the assembled nucleic acids are bound to the beads, wherein the beads are compartmentalized during clonal amplification such that the amplicons on each bead are separated from all of the amplicons on the other beads, so that amplicons cannot migrate from one bead to another bead; adding a bead-specific index sequence to free 3' ends of each clonally amplified assembled nucleic acid, and of amplicons thereof, using split-pool enzymatic DNA synthesis, to produce a plurality of indexed assembled nucleic acids bound to the beads, wherein the bead-specific index sequence for each bead comprises a unique identifier sequence that is different from the unique identifier sequence for the other beads; adding a reverse primer binding site to the free 3' ends of each indexed assembled nucleic acid bound to the beads using enzymatic DNA synthesis; amplifying the plurality of indexed assembled nucleic acids bound to the beads using a primer that hybridizes to the adapter primer binding site and a primer that hybridizes to the reverse primer binding site that was added by enzymatic DNA synthesis; sequencing at least a portion of the amplified indexed assembled nucleic acids to identify a correctly assembled nucleic acid comprising the sequence of interest; and selectively amplifying at least one correctly assembled nucleic acid comprising the sequence of interest using a primer that hybridizes to the bead-specific index sequence of at least one correctly assembled nucleic acid comprising the sequence of interest.

In some preferred embodiments, binding the plurality of assembled nucleic acids to beads is performed under conditions wherein no more than one assembled nucleic acid is bound to each bead. In some embodiments, binding the plurality of assembled nucleic acids to beads is performed with a nucleic acid-to-bead ratio ranging from 0.08 to 0.30.

In some embodiments, the method further comprises deep sequencing the amplified indexed assembled nucleic acids on one or more beads.

In some embodiments, the method further comprises using EDS to add a unique molecular identifier to assembled nucleic acids having different sequences.

In some embodiments, the beads are compartmentalized during clonal amplification by isolating each bead in separate emulsion droplets. In some embodiments, the beads are compartmentalized during clonal amplification by isolating each bead in separate compartments of a multi-compartment container.

In some embodiments, the method further comprises storing the amplified indexed assembled nucleic acids for a period of time prior to said performing nucleic acid amplification of the correctly assembled nucleic acid comprising the sequence of interest.

In some embodiments, the method further comprises analyzing sequences of the amplified indexed assembled nucleic acids to identify one or more additional correctly assembled nucleic acids comprising the sequence of interest; and identifying the bead-specific index sequences of the one or more additional correctly assembled nucleic acids comprising the sequence of interest; and performing nucleic acid amplification using primers having sequences that hybridize to the bead-specific index sequences of the one or more additional correctly assembled nucleic acids comprising the sequence of interest, wherein the one or more additional correctly assembled nucleic acids comprising the sequence of interest are selectively amplified.

In some embodiments, the adapters are linear adapters, Y-adapters, stubby adapters, or hairpin adapters. In some embodiment Y-adapters are used, wherein the double-stranded stems of the Y-adapters are ligated to the 5' and 3' ends of the plurality of assembled nucleic acids.

In some embodiments, the adapters further comprise a barcode.

In some embodiments, the method further comprises removing the adapters from the correctly assembled nucleic acid comprising the sequence of interest. In some embodiments, the assembled nucleic acids comprise a restriction site, wherein the ligated adapters can be removed by cleavage at the restriction site by an endonuclease.

In some embodiments, the method further comprises removing the unique identifier sequence from the correctly assembled nucleic acid comprising the sequence of interest.

In some embodiments, the plurality of assembled nucleic acids are assembled using an isothermal assembly method. In some embodiments, the isothermal method uses an exonuclease, a DNA polymerase, or a ligase, or any combination thereof. Exemplary assembly methods include, without limitation, Gibson assembly, polymerase chain assembly (PCA), Goldengate assembly, BioBrick assembly, or oligonucleotide ligation.

In some embodiments, performing clonal amplification comprises performing emulsion polymerase chain reaction.

In some embodiments, the beads are magnetic beads.

In some embodiments, the method further comprises preparing the plurality of assembled nucleic acids for ligation to the adapters by blunting the 3' and 5' ends of the assembled nucleic acids, phosphorylating the 5' ends of the assembled nucleic acids, or adding a poly A tail to the 3' ends of the assembled nucleic acids, or a combination thereof.

In some embodiments, prior to said ligating the adapters, the method further comprises: assembling a plurality of oligonucleotides into a plurality of polynucleotides to produce the plurality of assembled nucleic acids, wherein each oligonucleotide comprises a portion of the sequence of interest. In some embodiments, the oligonucleotides range from 20 base pairs to 400 base pairs in length, including any length within this range such as 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, or 400 base pairs in length. In some embodiments, the oligonucleotides are less than 300 base pairs in length, less than 200 base pairs in length, less than 150 base pairs in length, less than 100 base pairs in length, or less than 70 base pairs in length. In some embodiments, the oligonucleotides are more than 40 base pairs in length, more than 50 base pairs in length, more than 75 base pairs in length, more than 100 base pairs in length, or more than 200 base pairs in length. In some embodiments, the oligonucleotides have a length ranging from about 60 base pairs to about 80 base pairs, including any length within this range such as 60, 61, 62, 63, 64, 65, 66, 67, 68, 69, 70, 71, 72, 73, 74, 75, 76, 77, 78, 79, or 80 base pairs. In some embodiments, the oligonucleotides have a length ranging from 20 base pairs to 50 base pairs, including any length within this range such as 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 base pairs.

In some embodiments, the nucleic acid comprising a sequence of interest comprises an endogenous gene sequence encoding a protein or RNA, an exon, an intron, a wild-type sequence, a major allele, a minor allele, an allele linked to a disease, a nucleic acid comprising a mutation (e.g., deletion, insertion, substitution, single nucleotide polymorphism, frameshift mutation, missense mutation, or nonsense mutation), or an engineered nucleic acid.

In some embodiments, a next-generation sequencing (NGS) method is used to perform the sequencing. Exemplary NGS methods include, without limitation, second generation NGS technologies like those sold from Illumina or ION Torrent Technology from ThermoFisher, systems from Gynapsys, Omniome, Element Biosciences, Singular Genomics, and third generation NGS technologies such as single molecule real-time (SMRT) sequencing from Pacific Biosystems, and 4^{th} generation single molecule NGS technologies like nanopore sequencing from Oxford Nanopore.

In some embodiments, the sequencing comprises sequencing the adapter sequences, the bead-specific index sequences, and the assembled nucleic acid sequences.

In another non-claimed aspect, a system is described, the system comprising: a device for performing split-pool enzymatic DNA synthesis (EDS), a processor, wherein the processor is programmed to instruct the device to perform split-pool EDS to add (i) a bead-specific index sequence and (ii) a reverse priming site to free 3' ends of bead-bound clonally amplified assembled nucleic acids; and a sequencer, wherein the sequencer is connected to the processor such that the processor receives sequencing data from the sequencer.

In some aspects the processor is provided by a computer or handheld device (e.g., a cell phone or tablet).

In some aspects, the sequencer is inside the device for performing split-pool EDS.

In some aspects, the processor is further programmed to receive the sequence of a nucleic acid comprising a sequence of interest; design sequences of a plurality of polynucleotides that can be assembled into the nucleic acid comprising the sequence of interest, wherein each polynucleotide comprises a portion of the sequence of interest; and to instruct a synthesizer to synthesize the plurality of polynucleotides.

In some aspects, the system further comprises a chamber for assembly of the plurality of polynucleotides. In some aspects, the chamber further comprises an exonuclease, a DNA polymerase, and a ligase for performing the assembly of the plurality of polynucleotides.

In some aspects, the processor is further programmed to instruct the device to perform the assembly of the plurality of polynucleotides.

In some aspects, the system further comprises a memory storage component operably coupled to the processor, wherein the memory storage component is configured to store sequencing data.

In some aspects, the system further comprises a plurality of Y-adapters, a set of polymerase chain reaction (PCR) primers having sequences that are complementary to sequences in the 5' and 3' arms of the Y-adapters, a PCR primer having a sequence complementary to the reverse primer binding site, reagents for performing emulsion PCR, beads, or a ligase, or any combination thereof.

In some aspects, the system further comprises magnetic beads.

In some aspects, the system further comprises reagents for preparing the plurality of assembled nucleic acids for ligation to the adapters, wherein the reagents comprise reagents for blunting the 3' and 5' ends of the assembled nucleic acids, phosphorylating the 5' ends of the assembled nucleic acids, or adding a poly A tail to the 3' ends of the assembled nucleic acids, or a combination thereof.

In some aspects, the system further comprises reagents for performing gene assembly. In some aspects, the system comprises reagents for performing Gibson assembly, polymerase chain assembly, Goldengate assembly, BioBrick assembly, or oligonucleotide ligation.

In another non-claimed aspect, a kit is described, the kit comprising a system described herein, packaging for the system, and instructions for using the system for cell-free assembly of a nucleic acid comprising a sequence of interest and identification of a correctly assembled nucleic acid comprising the sequence of interest.

In another non-claimed aspect, a computer implemented method for assembly of a nucleic acid comprising a sequence of interest is described, the computer performing steps comprising: receiving a sequence of a nucleic acid to be assembled from a plurality of polynucleotides; designing sequences of the plurality of polynucleotides, wherein each polynucleotide comprises a portion of the sequence of the nucleic acid comprising the sequence of interest, wherein the plurality of polynucleotides can be assembled into the full-length sequence of the nucleic acid comprising the sequence of interest; instructing a synthesizer to synthesize the plurality of polynucleotides; instructing a device to add bead-specific index sequences and reverse priming sites to free 3' ends of a plurality of bead-bound clonally amplified assembled nucleic acids produced from the plurality of polynucleotides using split-pool enzymatic DNA synthesis (EDS), wherein the bead-specific index sequence for each bead comprises a different unique identifier sequence; receiving sequences of the indexed assembled nucleic acids; analyzing the sequences of the indexed assembled nucleic acids to identify the bead-specific index sequence of a correctly assembled nucleic acid comprising the sequence of interest; and displaying the bead-specific index sequence of the correctly assembled nucleic acid comprising the sequence of interest.

### BRIEF DESCRIPTION OF THE DRAWINGS

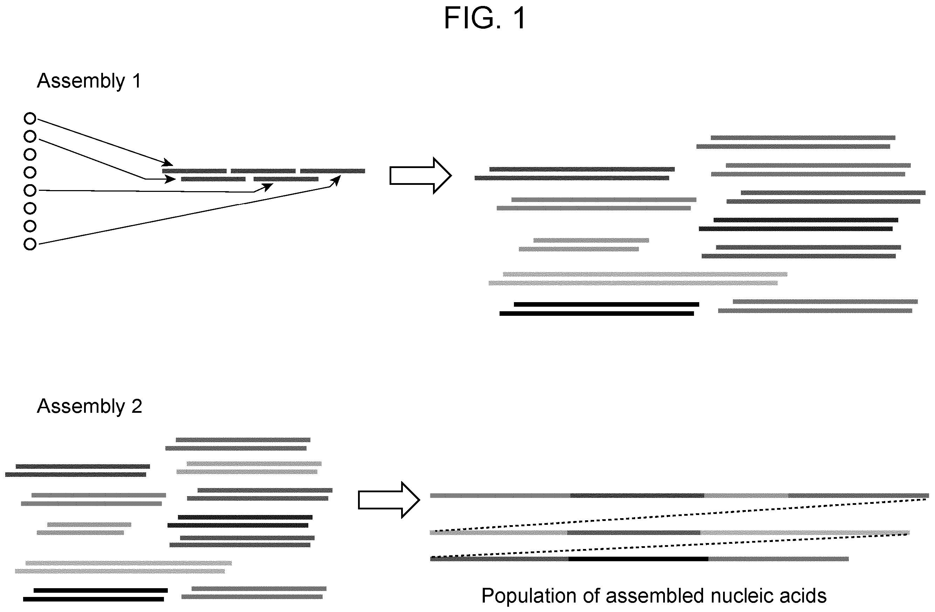

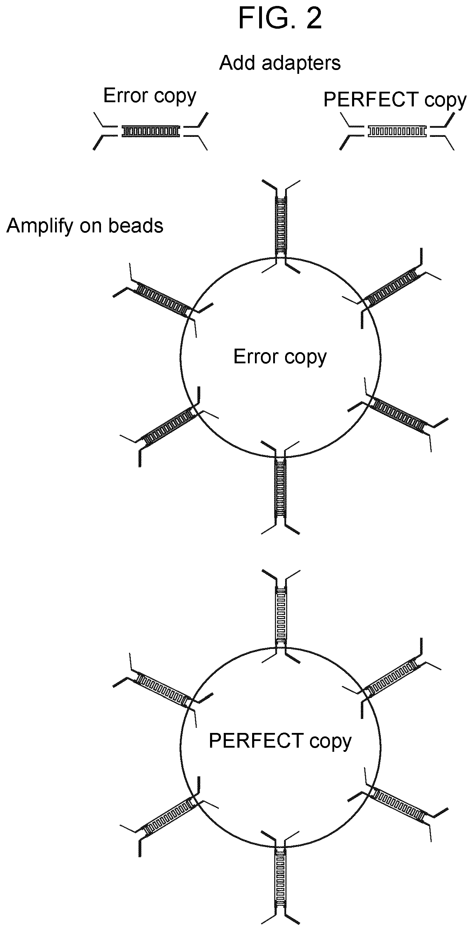

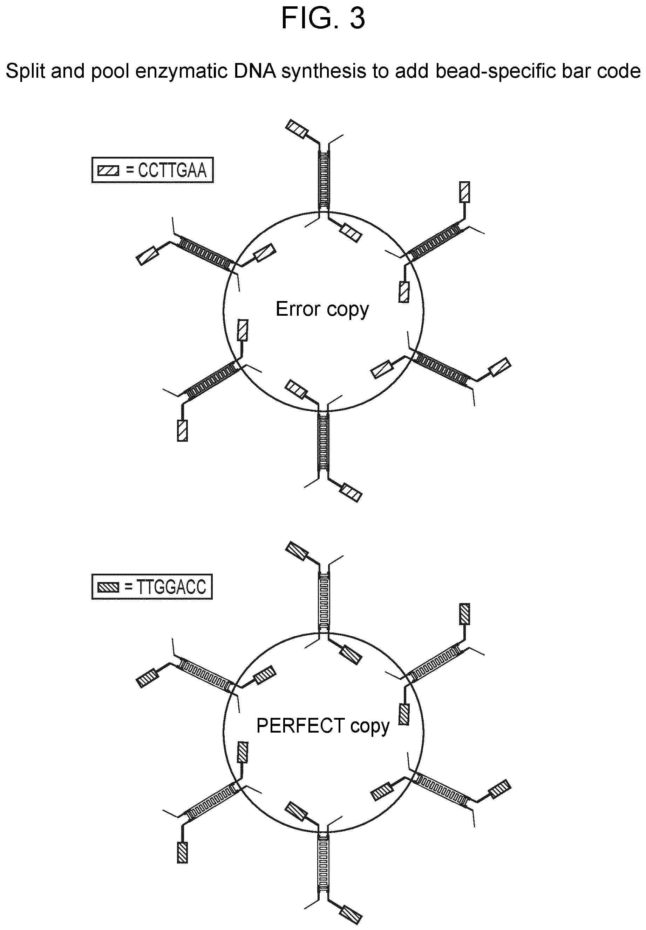

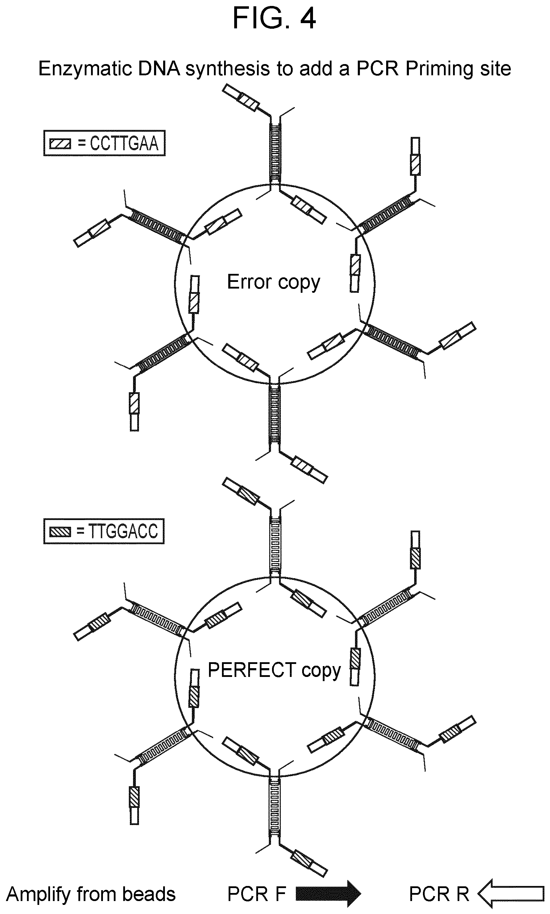

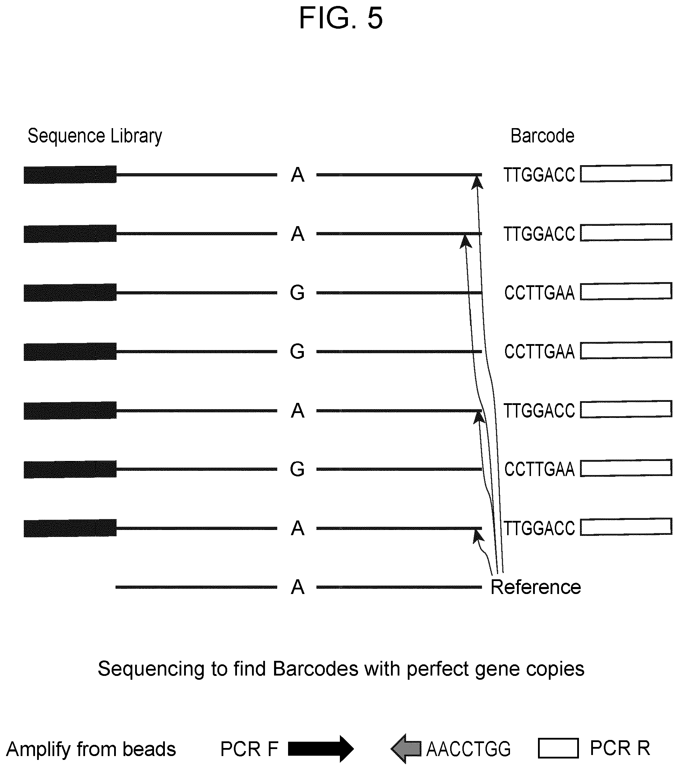

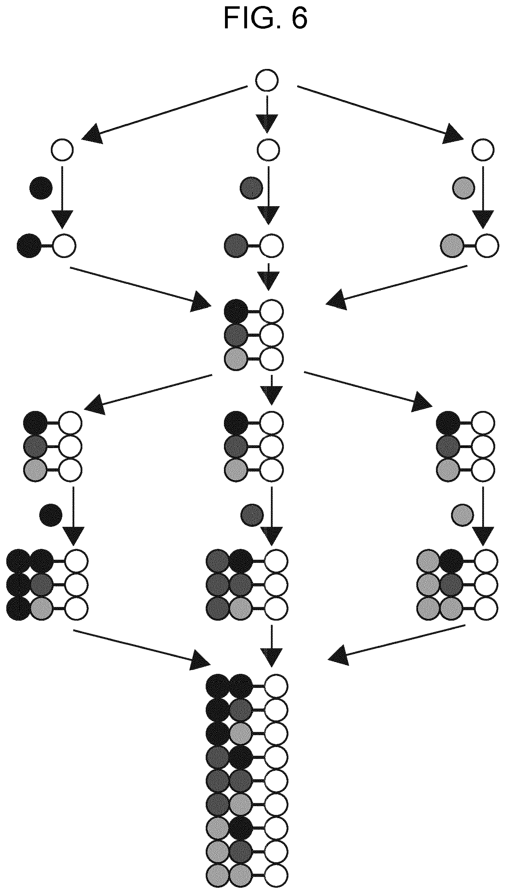

**FIGS. 1-5****.** Schematic overview of the protocol for cell-free identification of correctly assembled nucleic acids. **FIG. 1** shows assembly steps, including Assembly 1 (oligonucleotides into small fragments or exons) and Assembly 2 (small fragments into genes). **FIG. 2** shows adapter ligation and clonal amplification steps. **FIG. 3** shows the use of split and pool EDS to add bead-specific bar codes to assembled nucleic acids. **FIG. 4** shows the use of EDS to add a PCR priming site to indexed assembled nucleic acids. **FIG. 5** shows NGS sequencing to identify the bead-specific bar code of an assembled nucleic acid having the correct sequence and selective amplification with a primer complementary to that bead-specific bar code. **FIG.** 6 schematically depicts the split and pool approach, illustrated with the addition of three different alternatives after the splitting step and before the pooling step (adding at each step a light grey, a dark grey or a black circle to the growing chain constructed from the starting point, a white circle).

### DETAILED DESCRIPTION

Methods are provided, and systems and devices are described for cell-free identification and amplification of a correctly assembled nucleic acid comprising a sequence of interest from a mixture of nucleic acids. The subject methods use split-pool enzymatic DNA synthesis (EDS) to add a bead-specific index sequence to bead-bound clonally amplified assembled nucleic acids. The assembled nucleic acids are sequenced to identify one or more correctly assembled nucleic acids comprising the sequence of interest, wherein the bead-specific index sequence of each correctly assembled nucleic acid allows selective amplification of the correctly assembled nucleic acid out of a pooled sequencing library by using a primer having a sequence complementary to the bead-specific index sequence.

The present invention is not limited exactly to particular methods described herein, since some variations may also be practiced. Also, the terminology used herein is for the purpose of describing particular embodiments only, and is not intended to be limiting, since the scope of the present invention will be defined only by the granted claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art. Although any methods and materials similar or equivalent to those described herein can be used in the practice or testing of the present invention, some potential and preferred methods and materials are described.

### Definitions

The term "about", particularly in reference to a quantity, is meant to encompass deviations of plus or minus five percent.

The terms "polynucleotide" "oligonucleotide", "nucleic acid", and "nucleic acid molecule" are used herein to include polymeric forms of nucleotides of any length, such as ribonucleotides and/or deoxyribonucleotides. Thus, these terms may be used to refer to triple-, double- and single-stranded forms of DNA, as well as triple-, double- and single-stranded forms of RNA. These terms also include modifications, such as by methylation and/or by capping, and unmodified forms. More particularly, the terms "polynucleotide", "oligonucleotide", "nucleic acid", and "nucleic acid molecule" include polydeoxyribonucleotides (containing 2-deoxy-D-ribose), polyribonucleotides (containing D-ribose), any other type of polynucleotide which is an N- or C-glycoside of a purine or pyrimidine base, and other polymers containing non-nucleotidic backbones, for example, polyamide (e.g., peptide nucleic acids (PNAs)) and polymorpholino (commercially available from the Anti-Virals, Inc., Corvallis, Oregon, as Neugene) polymers, and other synthetic sequence-specific nucleic acid polymers providing that the polymers contain nucleobases in a configuration which allows for base pairing and base stacking, such as is found in DNA and RNA. There is no intended distinction in length between the terms "polynucleotide", "oligonucleotide", "nucleic acid", and "nucleic acid molecule," and these terms will be used interchangeably, except when they have particular meanings based on their context of use. Thus, these terms include, for example, 3'-deoxy-2',5'-DNA, oligodeoxyribonucleotide N3' P5' phosphoramidates, 2'-O-alkyl-substituted RNA, double- and single-stranded DNA, as well as double- and single-stranded RNA, DNA:RNA hybrids, and hybrids between PNAs and DNA or RNA, and also include known types of modifications, for example, labels which are known in the art, methylation, "caps," substitution of one or more of the naturally occurring nucleotides with an analog, internucleotide modifications such as, for example, those with uncharged linkages (e.g., methyl phosphonates, phosphotriesters, phosphoramidates, carbamates, etc.), with negatively charged linkages (e.g., phosphorothioates, phosphorodithioates, etc.), and with positively charged linkages (e.g., aminoalklyphosphoramidates, aminoalkylphosphotriesters), those containing pendant moieties, such as, for example, proteins (including nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide. In particular, DNA is deoxyribonucleic acid.

As used herein, the term "target nucleic acid" denotes a nucleic acid molecule that contains a "target sequence" to be amplified. The target nucleic acid may be single-stranded or double-stranded and may include additional nucleotides on one or both sides of the target sequence. The term "target sequence" refers to the particular nucleotide sequence of the target nucleic acid which is to be amplified. In some embodiments, the target sequence includes a probe-hybridizing region with which a probe will form a stable hybrid under suitable hybridization conditions. The "target sequence" may also include sequences that are complementary to one or more primers that can be extended by a polymerase or ligase using the target sequence as a template. If the target nucleic acid is originally single-stranded (e.g., a "sense strand"), the term "target sequence" also refers to the sequence complementary to the "target sequence" as present in the target nucleic acid. If the "target nucleic acid" is originally double-stranded, the term "target sequence" refers to both the plus (+) and minus (-) strands (or sense and anti-sense strands).

The term "primer" or "oligonucleotide primer" as used herein, refers to an oligonucleotide that hybridizes to a template strand of a nucleic acid and initiates synthesis of a nucleic acid strand complementary to the template strand in the presence of nucleotides and a polymerization-inducing agent such as a DNA or RNA polymerase and under suitable temperature, pH, metal concentration, and salt conditions. The primer is usually single-stranded, but may alternatively be double-stranded or contain a double-stranded portion. If double-stranded, the primer can first be treated to separate its strands before being used to form primer extension products. This denaturation step is typically effected by heat, but may alternatively be carried out using alkali, followed by pH neutralization. Thus, a "primer" is complementary to a template sequence, and hybridization complexes by hydrogen bonding or hybridization with the template to give a primer/template complex for initiation of primer extension by a polymerase, to add nucleotides to the 3' end of the primer that are complementary to the corresponding template sequence. Typically, nucleic acids are amplified using at least one forward primer and at least one reverse primer capable of hybridizing to regions of a nucleic acid flanking the portion of the nucleic acid sequence to be amplified. The term "amplicon" refers to the amplified nucleic acid product of a PCR reaction or other nucleic acid amplification process that creates copies of a single or double stranded nucleic acid sequence (e.g., rolling circle amplification or isothermal amplification methods such as recombinase polymerase amplification (RPA), loop-mediated isothermal amplification (LAMP), strand displacement amplification (SDA), helicase-dependent amplification (HDA), and nicking enzyme amplification reaction (NEAR), and the like). Amplicons may comprise RNA or DNA depending on the technique used for amplification.

The terms "hybridize" and "hybridization" refer to the formation of complexes between nucleotide sequences which are sufficiently complementary to form complexes via Watson-Crick base pairing. Where a primer "hybridizes" with target (template), such complexes (hybrids) are sufficiently stable to serve the priming function required by, e.g., a DNA polymerase to initiate DNA synthesis. It will be appreciated that sequences need not have perfect complementarity to provide stable hybrids. In many situations, stable hybrids can form where fewer than about 10% of the bases are mismatches, ignoring loops of four or more nucleotides. Accordingly, as used herein the term "complementary" refers to an oligonucleotide that forms a stable duplex with its "complement" under suitable conditions, often where there is about 90% or greater homology.

The "melting temperature" or "Tm" of double-stranded DNA is defined as the temperature at which half of the helical structure of DNA is lost due to heating or other dissociation of the hydrogen bonding between base pairs, for example, by alkali treatment, or the like. The Tₘ of a DNA molecule depends on its length and on its base composition. DNA molecules having high GC base pair content have a higher Tₘ than those having less GC base pair content. Separated complementary strands of DNA spontaneously reassociate or anneal to form duplex DNA when the temperature is lowered below the Tₘ. The Tₘ may be estimated using the following relationship: Tₘ = 69.3 + 0.41(GC)% (Marmur et al. (1962) J. Mol. Biol. 5:109-118).

The term "Y-adapter" refers to an adapter that has a Y-shaped structure comprising two partially complementary strands hybridized to each other, such that the adapter has a stem region and two single-stranded arms. Each of the two strands in a Y-adapter has a 5' end region and a 3' end region, such that the 5' end region of a first strand is complementary to and hybridizes to the 3' end region of the other (second) strand to form a double-stranded stem region (the "stem"). The 3' end region of the first strand and the 5' end region of the second strand provide single-stranded 3' and 5' arms since they are non-complementary to each other and therefore do not hybridize substantially to each other. Y-adapters can be attached to double-stranded nucleic acids comprising a target sequence of interest by ligating the free 3' and 5' ends of the complementary strand regions of the double-stranded stem of the Y-adapter to the 5' and 3' ends of each nucleic acid. The use of Y adapters allows the addition of different adapter sequences to the 5' and 3' ends of double-stranded nucleic acids in a DNA library simultaneously.

The terms "hairpin adapter", "loop adapter", and "hairpin loop adapter" refer to an adapter that comprises a hairpin loop structure. After ligation of a hairpin adapter to the end or ends of a double strand nucleic acid, the hairpin loop can be cleaved to produce strands that have non-complementary sequences on the ends, thereby generating a Y-adapter having a single-stranded 5' arm and a single-stranded 3' arm. In some cases, the loop of a hairpin adapter may contain a uracil residue, wherein the loop can be cleaved using uracil DNA glycosylase and endonuclease VIII.

The terms "connected" or "coupled" are used in an operational sense and are not necessarily limited to a direct connection or coupling. For example, two devices or components may be coupled directly, or via one or more intermediary media or devices. As another example, devices may be coupled in such a way that information or data can be passed between them, while not sharing any physical connection with one another. In some cases, two devices or components may be connected by a wire or wirelessly to each other.

### Cell-Free Methods for Identification and Amplification of a Correctly Assembled Nucleic Acid

The methods, systems, and devices described herein allow cell-free identification and amplification of a correctly assembled nucleic acid, or of one or more correctly assembled nucleic acids, comprising a sequence of interest from a mixture of nucleic acids. The subject methods generally comprise: ligating adapters to the 5' and 3' ends of a plurality of assembled nucleic acids, wherein the adapters comprise an adapter primer binding site and an adapter capture sequence; binding the plurality of assembled nucleic acids to beads, wherein the beads comprise a capture oligonucleotide attached to the surface of the beads that is complementary to and specifically hybridizes to the adapter capture sequence; performing clonal amplification of the plurality of assembled nucleic acids bound to the beads using (i) the capture oligonucleotide attached to the bead as a first primer and (ii) a second primer that hybridizes to the adapter primer binding site, wherein amplicons produced by clonal amplification of the assembled nucleic acids are bound to the beads, wherein the beads are compartmentalized during clonal amplification such that the amplicons on each bead are separated from the other amplicons on all other beads so that the amplicons cannot migrate from one bead to another bead; adding a bead-specific index sequence to free 3' ends of each clonally amplified assembled nucleic acid, and of amplicons thereof, using split-pool enzymatic DNA synthesis (EDS) to produce a plurality of indexed assembled nucleic acids bound to the beads, wherein the bead-specific index sequence for each bead comprises a different unique identifier sequence; adding a reverse priming site to the free 3' ends of each indexed assembled nucleic acid bound to the beads using EDS; amplifying the plurality of indexed assembled nucleic acids bound to the beads using a primer that hybridizes to the adapter primer binding site and a primer that hybridizes to the reverse priming site added by EDS; sequencing at least a portion of the amplified indexed assembled nucleic acids to identify a correctly assembled nucleic acid comprising the sequence of interest; identifying the bead-specific index sequence of the correctly assembled nucleic acid comprising the sequence of interest; and performing nucleic acid amplification using a primer that hybridizes to the bead-specific index sequence of the correctly assembled nucleic acid comprising the sequence of interest, wherein the correctly assembled nucleic acid comprising the sequence of interest is selectively amplified.

In some embodiments, the beads are compartmentalized during clonal amplification by isolating each bead in separate emulsion droplets or in separate compartments of a multi-compartment container.

In some embodiments, the method further comprises using enzymatic DNA synthesis to add a unique molecular identifier to assembled nucleic acids having different sequences.

In some embodiments, the method further comprises storing the amplified indexed assembled nucleic acids for a period of time prior to performing nucleic acid amplification of the correctly assembled nucleic acid comprising the sequence of interest.

In some embodiments, the method further comprises analyzing sequences of the amplified indexed assembled nucleic acids to identify one or more additional correctly assembled nucleic acids comprising the sequence of interest; and identifying the bead-specific index sequences of the one or more additional correctly assembled nucleic acids comprising the sequence of interest; and performing nucleic acid amplification using primers having sequences that are complementary to and hybridize to the bead-specific index sequences of the one or more additional correctly assembled nucleic acids comprising the sequence of interest, wherein the one or more additional correctly assembled nucleic acids comprising the sequence of interest are selectively amplified.

In some embodiments, prior to ligating the adapters, the method further comprises producing a plurality of assembled nucleic acids. Various methods can be used for assembly of a nucleic acid from a plurality of nucleic acid fragments comprising a portion of the sequence of interest, as described further below. In some cases, a nucleic acid comprising a sequence of interest is assembled from multiple oligonucleotides each comprising a different portion of the sequence of interest. In some embodiments, the oligonucleotides used to assemble a nucleic acid range from 20 base pairs to 400 base pairs in length, including any length within this range such as 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, or 400 base pairs in length. In some embodiments, the oligonucleotides are less than 300 base pairs in length, less than 200 base pairs in length, less than 150 base pairs in length, less than 100 base pairs in length, or less than 70 base pairs in length. In some embodiments, the oligonucleotides are more than 40 base pairs in length, more than 50 base pairs in length, more than 75 base pairs in length, more than 100 base pairs in length, or more than 200 base pairs in length. In some embodiments, the oligonucleotides have a length ranging from about 60 base pairs to about 80 base pairs, including any length within this range such as 60, 61, 62, 63, 64, 65, 66, 67, 68, 69, 70, 71, 72, 73, 74, 75, 76, 77, 78, 79, or 80 base pairs. In some embodiments, the oligonucleotides have a length ranging from 20 base pairs to 50 base pairs, including any length within this range such as 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 base pairs.

The beads used to bind the assembled nucleic acids may be magnetic beads (e.g., superparamagnetic beads) or non-magnetic beads. Magnetic beads have the advantage of allowing isolation of bound nucleic acids by magnetic separation techniques. In some embodiments, the beads further comprise a coating. For example, the surface of a bead may be coated with silica or functionalized to facilitate binding of the assembled nucleic acids. In some embodiments, capture oligonucleotides are attached to the surface of a bead, wherein the capture oligonucleotides have a sequence that is complementary to a universal adapter sequence or a sequence present in all or a subset of the assembled nucleic acids. Using the capture oligonucleotide as a primer for amplification of an assembled nucleic acid allows a newly synthesized strand to become covalently attached to the bead, thus immortalizing the strand and creating a resource which can support amplification of the perfect copies at ones convenience.

Binding of the assembled nucleic acids to beads is preferably performed under suitable conditions to allow no more than one assembled nucleic acid to bind to each bead. A solution containing the assembled nucleic acids can be diluted to provide a nucleic acid-to-bead ratio, wherein no more than one assembled nucleic acid is bound per bead. If the nucleic acid-to-bead ratio is too high, some of the beads will have multiple different assembled nucleic acids bound to them. If the nucleic acid-to-bead ratio is too low, many of the beads will not have any assembled nucleic acids bound to them. The nucleic acid-to-bead ratio may be optimized such that most of the beads contain a single bound assembled nucleic acid. In some embodiments, the nucleic acid-to-bead ratio is in the range of 0.08 to 0.30, including any ratio within this range such as 0.08, 0.081, 0.082, 0.083, 0.084, 0.085, 0.09, 0.095, 0.1, 0.2, or 0.3. In some embodiments, the nucleic acid-to-bead ratio ranges from 0.08 to 0.09.

Adapters and index sequences can be removed from the final correctly assembled nucleic acid product by various methods. For example, PCR can be performed with primers designed to only amplify the sequence of interest without the other added sequences. Alternatively, a restriction site can be added to allow the adapters to be cleaved from the sequence of interest by a restriction enzyme. In cases in which Gibson assembly is used, the adapters can be eliminated by using them as a target for removal by recombination with a plasmid.

The nucleic acid to be assembled by the methods described herein can comprise any type of sequence of interest. In some embodiments, the assembled nucleic acid comprises an endogenous gene encoding a protein or RNA, an exon, an intron, a wild-type sequence, a major allele, a minor allele, an allele linked to a disease, a nucleic acid comprising a mutation (e.g., a deletion, insertion, substitution, single nucleotide polymorphism, frameshift mutation, missense mutation, or nonsense mutation), or an engineered nucleic acid.

### Assembly

Assembly of a nucleic acid is typically performed in at least two steps: first, short DNA fragments (e.g., oligonucleotides) comprising portions of the desired nucleic acid sequence are assembled into longer DNA fragments (e.g., partially assembled nucleic acid intermediates, which for this example can also be referred to as "polynucleotides"); next, the longer DNA fragments are assembled to generate the complete nucleic acid. In some cases, assembly of a nucleic acid comprises multiple steps with production of multiple partially assembled nucleic acid intermediates having progressively increasing length at each assembly step. DNA fragments (e.g., oligonucleotides or polynucleotides) with overlapping sequences can be connected by annealing and ligation and/or polymerase-mediated reactions. For example, DNA fragments can be ligated together using a ligase in the absence of any template, as normally required for DNA polymerase. Endonucleases, which cleave nucleic acids into fragments, can be used to direct DNA assembly. Any suitable method for assembling a nucleic acid from multiple DNA fragments can be used to produce a complete nucleic acid comprising a sequence of interest. Exemplary methods for nucleic acid assembly from DNA fragments include, without limitation, Gibson assembly, polymerase chain assembly (PCA), Goldengate assembly, BioBrick assembly, and oligonucleotide ligation.

Gibson assembly can be used to combine DNA fragments under isothermal conditions using an exonuclease, a DNA polymerase, and a ligase. Adjacent DNA fragments must contain an overlap region (typically about 20-40 base pairs). The overlap regions can be added to adjacent DNA fragments, for example, using PCR or EDS. First, a 5' exonuclease is used to cleave DNA from the 5' ends of DNA fragments to generate single-stranded overhangs. Next, the single-stranded regions on adjacent DNA fragments are annealed, and a DNA polymerase is used to add nucleotides to fill in any gaps in the annealed single-stranded regions. Then, a DNA ligase is used to covalently join the adjacent DNA segments and to seal any nicks in the annealed DNA. Gibson assembly has advantages over conventional cloning methods that no restriction digest of the DNA fragments after PCR is necessary. For a description of Gibson assembly, see, e.g., Gibson et al. (2009) Nat. Methods 6:343-345, Gibson et al. (2010) Science 329, 52-56.

Polymerase chain assembly (also known as polymerase cycling assembly or PCA) is similar to PCR but uses multiple single-stranded overlapping oligonucleotides that anneal to one another. Each oligonucleotide is designed to be either part of the sense strand or anti-sense strand of the double-stranded target DNA to be assembled. The single-stranded oligonucleotides are designed to cover most of the sequences of both strands of the full-length nucleic acid sequences to be assembled. The overlapping terminal regions of the oligonucleotides must have sufficient complementarity to assemble into the final complete sequence. Preferably, the complementary overlapping terminal regions have similar melting temperatures when annealed to their complementary counterparts, are hairpin-free, and are not too GC-rich. A forward primer and a reverse primer are used for priming to allow a DNA polymerase to fill in the entire template sequence. The primers used for PCA are longer than for PCR and typically have lengths of 40 to 50 nucleotides to ensure correct hybridization. During PCA cycles, the oligonucleotides anneal to complementary oligonucleotides and then are extended in a template-dependent manner by the polymerase until the 5'-end of the template strand is reached. Each cycle increases the length of the various oligonucleotides that anneal to each other. Forward and reverse PCR primers are used to selectively amplify the complete target sequence out of the product mixture, which will also contain shorter incomplete fragments. Gel electrophoresis or chromatography can then be used to isolate the complete target sequence. A typical reaction consists of oligonucleotides having a length of about 40 to 50 base pairs, wherein each oligonucleotide has an overlapping region of about 20 base pairs. The PCA reaction is carried out with all the oligonucleotides, typically for about 30 cycles, followed by about 23 additional cycles with the PCR primers that selectively amplify the desired assembled complete DNA product. For a description of PCA, see, e.g., Stemmer; et al. (1995) Gene 164(1):49-53, Smith et al. (2003) Proc. Natl. Acad. Sci. USA 100(26):15440-15445, Marchand et al. (2012) Methods Mol. Biol. 852:3-10.

Goldengate assembly uses Type IIS restriction enzymes and DNA ligase (e.g., T4 DNA ligase) to directionally assemble multiple DNA fragments into a complete DNA construct. For Goldengate assembly, type IIS restriction enzymes that create 4-base overhangs are commonly used. Exemplary restriction enzymes that may be used in Goldengate assembly include, without limitation, BsaI, BsmBI, BsmBI-v2, PaqCI, BsaI-HF, BbsI, BbsI-HF, and Esp3I. The Type IIS restriction enzymes chosen should cut the DNA outside of their recognition sites to create non-palindromic overhangs. Using such Type IIs restriction enzymes allows Golden Gate assembly to be scarless because the final ligated product does not retain the Type IIS restriction enzyme recognition site and cannot be cut again by the restriction enzyme used for assembly. The Type IIS restriction enzymes are selected such that combinations of overhang sequences are generated that allow multiple DNA fragments to be assembled directionally, which can then be ligated together to generate the final assembled DNA product. In some case, multiple DNA fragments are generated with the same Type IIS restriction enzyme. For example, BsaI can create 256 different four-base pair overhangs depending on the sequence of the DNA. Overhangs of DNA fragments should be designed so that the DNA fragments can be ligated without scar sequences between them. Vectors used in Goldengate assembly contain the DNA fragments used in assembly flanked by the Type IIS restriction enzyme recognition sites. After cutting with the Type IIS restriction enzymes, each DNA fragment used in the assembly has unique overhangs that anneal to the next DNA fragment and become ligated to produce the complete assembled DNA. The sequence of the DNA fragment overhangs allows assembly of multiple fragments simultaneously in the correct order to generate the final assembled DNA product. Because restriction sites are not present in the final ligated product, digestion and ligation can be carried out simultaneously. Variations of Goldengate assembly include MoClo and Golden Braid assembly, which entail multiple tiers of DNA assembly from parts constructed at each round of assembly. These methods can be used to assemble multi-gene or multi-part constructs with multiple transcription units. For a description of Goldengate assembly, and variations of it, see, e.g., Engler, et al. (2014) "Golden Gate cloning" Methods in Molecular Biology1116:119-131, Engler et al. (2008) PLoS ONE 3:e3647, Engler, C., et al. (2009) PLoS ONE 4:e5553, Weber et al. (2011) PLOS ONE 6 (2): e16765 Lee et al, (1996) Genet. Anal. 13(6):139-145, Padgett et al. (1996) Gene 168(1):31-35, Werner, S., et al. (2012) Bioeng Bugs 3(1):38-43, Andreou et al. (2018) PLoS ONE 13(1):e0189892, Pryor et al. (2020) PLOS ONE. 15 (9): e0238592, Wu et al. (2018) Mol. Plant Pathol. 19(6):1511-1522, Moore et al. (2016) ACS Synthetic Biology. 5 (10):1059-1069, Crozet et al. (2018) ACS Synthetic Biology. 7 (9): 2074-2086.

BioBrick assembly uses DNA sequences which conform to a restriction-enzyme assembly standard. Each BioBrick part has a DNA sequence contained in a circular plasmid. The BioBrick part is flanked by universal upstream and downstream sequences comprising restriction sites for specific restriction enzymes, at least two of which are isocaudomers. The BioBrick parts can be linked together in any desired order by removal of the restriction sites between the parts using restriction enzymes that cleave at the recognition sites. The BioBrick parts do not contain any of the restriction sites in the universal upstream and downstream sequences. BioBrick assembly can be used for assembly of any DNA molecule but is especially well suited for applications in synthetic biology to combine pieces of DNA with different functions. For a description of BioBrick standards and assembly methods, see, e.g., Knight (2003). Idempotent Vector Design for Standard Assembly of Biobricks" (hdl:1721.1/21168), Knight et al. (2008) J. Biol. Eng. 2:5, Shetty, et al. (2008). J Biol Eng. 2008 2:5, Shetty et al. (2011) Methods Enzymol. 498:311-326, Zucca et al. (2013) J. Biol. Eng. 7(1):12, Yamazaki et al. (2017) Synth Biol (Oxf) 2(1):ysx003, Smolke et al. (2009) Nature Biotechnology. 27 (12):1099-1102, Sleight et al. (2010) Nucleic Acids Research. 38 (8): 2624-2636, and Rokke et al. (2014) "BioBrick Assembly Standards and Techniques and Associated Software Tools" Methods in Molecular Biology Vol. 1116, Humana Press pp. 1-24.

### Adapters

Adapter oligonucleotides comprising known sequences are added to the 5' and 3' ends of nucleic acids to facilitate amplification and/or sequencing. Adapters can be designed with a primer binding site having a sequence suitable for hybridizing to primers for primer-dependent amplification and/or sequencing, Adapters may also include sites that allow nucleic acids to attach to a solid support. To facilitate multiplexing, the adapters can be barcoded.

Adapter oligonucleotides can be ligated to the ends of a nucleic acid using a ligase. Any suitable ligase may be used, including, without limitation, phage ligases such as T4 or T7 DNA ligase, archaeal ligases, or bacterial ligases. The adapters that are ligated to either end of a nucleic acid may be the same or different. Adapters may be attached to the ends of DNA by either blunt end ligation or sticky end ligation. Ligation of the ends of two DNA molecules involves formation of a phosphodiester bond between the 3'-hydroxyl group at the 3' end of one DNA molecule with the 5'-phosphoryl group at the 5' end of another DNA molecule. The ends of DNA molecules may be prepared for ligation by blunting of the DNA ends and phosphorylation of the 5' end. Blunting involves removing a single-stranded overhang (e.g., which may have been created by a restriction enzyme) by adding nucleotides to the complementary strand using the overhang as a template for polymerization, or removing the overhang using an exonuclease. DNA ends may be "blunted" to allow non-compatible ends to be joined by ligation. DNA polymerases, such as the Klenow fragment of DNA polymerase I and T4 DNA polymerase can be used to fill in nucleotides or chew back a 3' overhang. A nuclease, such as Mung Bean Nuclease may be used for removal of a 5' overhang. In some cases, adapters are designed with a poly T overhang, which allows an adapter to be ligated to the 3'-ends of DNA having a poly A-overhang. For example, the ends of nucleic acids may be blunted and phosphorylated at the 5' ends by treating the DNA with T4 polynucleotide kinase, T4 DNA polymerase, and the Klenow large fragment. A poly A tail can be added to the 3' ends of nucleic acids using either Taq polymerase or the Klenow large fragment.

Solid phase amplification of polynucleotides is typically performed by first ligating known adapter sequences to each end of a target polynucleotide. The double-stranded polynucleotide is then denatured to form a single-stranded template molecule that is immobilized on a solid support (e.g., the surface of a flow-cell for the Illumina platform or beads for the Ion Torrent platform). The adapter sequence on the 3' end of the template is hybridized to an extension primer, and amplification is performed by extending the primer. In some aspects, a sequencing platform adapter construct includes one or more nucleic acid domains selected from: a domain (e.g., a "capture site" or "capture sequence") that specifically binds to a surface-attached sequencing platform oligonucleotide (e.g., the P5 or P7 oligonucleotides attached to the surface of a flow cell in an Illumina^{®} sequencing system); a sequencing primer binding domain (e.g., a domain to which the Read 1 or Read 2 primers of the Illumina^{®} platform may bind); a barcode domain (e.g., a domain that uniquely identifies the sample source of the nucleic acid being sequenced to enable sample multiplexing by marking every molecule from a given sample with a specific barcode or "tag"); a barcode sequencing primer binding domain (a domain to which a primer used for sequencing a barcode binds); or any combination of such domains.

The adapters chosen for the preparation of a sequencing library should be compatible with the sequencing system to be used. Polynucleotides are incorporated into the sequencing library by ligation to sequencing adapters containing specific sequences designed to work with the sequencing platform (i.e., sequencing platform adapter domain). A sequencing platform adapter domain, when present in an adapter, may include one or more nucleic acid domains of any length and sequence suitable for the sequencing platform of interest. In some embodiments, the nucleic acid domains are from 4 to 200 nucleotides in length. For example, the nucleic acid domains may be from 4 to 100 nucleotides in length, such as from 6 to 75, from 8 to 50, or from 10 to 40 nucleotides in length. According to some embodiments, the sequencing platform adapter construct includes a nucleic acid domain that is from 2 to 8 nucleotides in length, such as from 9 to 15, from 16 to 22, from 23 to 29, or from 30 to 36 nucleotides in length.

The nucleic acid domains may have a length and sequence that enables a polynucleotide (e.g., an oligonucleotide) employed by the sequencing platform of interest to specifically bind to the nucleic acid domain, e.g., for solid phase amplification and/or sequencing. Example nucleic acid domains include the P5 (5'-AATGATACGGCGACCACCGA-3') (SEQ ID NO: 1), P7 (5'-CAAGCAGAAGACGGCATACGAGAT-3') (SEQ ID NO:2), Read 1 primer (5'-ACACTCTTTCCCTACACGACGCTCTTCCGATCT-3') (SEQ ID NO:3) and Read 2 primer (5'-GTGACTGGAGTTCAGACGTGTGCTCTTCCGATCT-3') (SEQ ID NO:4) domains employed on the Illumina^{®}-based sequencing platforms. Other example nucleic acid domains include the A adapter (5'-CCATCTCATCCCTGCGTGTCTCCGACTCAG-3') (SEQ ID NO:5) and P1 adapter (5'-CCTCTCTATGGGCAGTCGGTGAT-3') (SEQ ID NO:6) domains employed on the Ion Torrent^{™}-based sequencing platforms. The nucleotide sequences of nucleic acid domains useful for sequencing on a sequencing platform of interest may vary and/or change over time. Adapter sequences are typically provided by the manufacturer of the sequencing platform (e.g., in technical documents provided with the sequencing system and/or available on the manufacturer's website). Based on such information, the sequence of any sequencing platform adapter domains, amplification primers, and/or the like, may be designed to include all or a portion of one or more nucleic acid domains in a configuration that enables sequencing the nucleic acid insert (corresponding to the assembled nucleic acid product) on the platform of interest.

Any suitable type of adapter may be ligated to the assembled nucleic acids, including, without limitation, linear adapters, Y adapters, stubby adapters, and hairpin adapters. In some embodiments, two different linear adapters are ligated to the 5' and 3' ends of every member of a library. A disadvantage of using linear double stranded adapters is that if two different adapters (referred to as A and B) are ligated to double-stranded DNA in a library, a mixture of ligation products is generated including about 50% incorrectly ligated products with the same adapter on both ends (A-insert-A, B-insert-B), and only 50% having correctly ligated adapters (e.g., A-insert-B or B-insert-A). The DNA inserts having A-A and B-B adapters are unable to be amplified by PCR.

Y-adapters have a Y-shaped structure with a single-stranded 5' arm, a single stranded 3' arm, and a double-stranded stem. The arms of the Y have noncomplementary sequences, and the stem is double-stranded with complementary strands of DNA that hybridize to each other. Y-adapters are attached to the assembled nucleic acids by ligating the double-stranded stem of the Y-adapters to the 5' and 3' ends of the assembled nucleic acids. The use of Y adapters allows the addition of different adapter sequences to the 5' and 3' ends of a DNA library simultaneously. An advantage of using Y adapters rather than linear adapters is that the Y adapters provide an efficient method of attaching two different adapter sequences on the 5' and 3' ends of every assembled nucleic acid in a library with a single adapter. After ligation of a Y adapter to both DNA ends, the library can be amplified using primers having sequences that are complementary to the sequences of the 5' and 3' arms of the Y-adapters. The use of Y adapters also allows both DNA strands to be sequenced.

An exemplary Y-adapter compatible with the Ion Torrent sequencing platform comprises: a 5' arm comprising the nucleotide sequence of CCATCTCATCCCTGCGTGTCTCCGACTCAG (SEQ ID NO:7), which includes a primer binding site comprising the nucleotide sequence of CCATCTCATCCCTGCGTGTCTCCGAC (SEQ ID NO:8), a sequence key (TCAG) used for recognition of library molecules and signal normalization, and a barcode sequence with a barcode end signal (GAT); a 3' arm comprising the nucleotide sequence of GGAGAGATACCCGTCAGCCACTATCTGA (SEQ ID NO:9), which includes a primer binding site comprising the nucleotide sequence of GGAGAGATACCCGTCAGCCACTA (SEQ ID NO:10); and a stem comprising a first DNA strand comprising the nucleotide sequence of AGCACGAATCGAT (SEQ ID NO:11) and a second DNA strand comprising the complementary nucleotide sequence of TCGTGCTTAGCT SEQ ID NO:12), as described by Forth et al. (BioTechniques (2019) 67:229-237. An exemplary Y-adapter compatible with the Illumina sequencing platform comprises one strand comprising the nucleotide sequence of AATGATACGGCGACCACCGAGATCTACACTCTTTCCCTACACGACGCTCTTCCGA TCT (SEQ ID NO:13) and a second strand comprising the nucleotide sequence of GATCGGAAGAGCACACGTCTGAACTCCAGTCACNNNNNNATCTCGTATGCCGTC TTCTGCTTG (SEQ ID NO:14), which includes a six nucleotide index sequence (NNNNNN), shown underlined, that varies in sequence. The xGen Stubby adapter from Integrated DNA Technologies, Inc. (Coralville, Iowa) is a short Y adapter that can be ligated to fragments with A overhangs and used with proprietary unique dual indexing (UDI) primer pairs.

In some embodiments, a hairpin adapter is used. Hairpin adapters generally comprise a double-stranded "stem" region and a single stranded "loop" region. In some embodiments, the hairpin adapter comprises one strand (i.e., one continuous strand) capable of adopting a hairpin structure, wherein the hairpin adapter comprises a self-complementary palindromic region that forms the stem and a non-complementary region that forms the loop of the hairpin adapter. In addition, hairpin adapters may comprise various components of adapters as described herein, including, without limitation, amplification priming sites, barcode sequences, and specific sequencing platform adapter domain sequences (e.g., P5 and P7 or A and P1 adapter sequences). Hairpin adapters may further comprise one or more cleavage sites capable of being cleaved under cleavage conditions. In some embodiments, a cleavage site is located in the loop region. Cleavage at a cleavage site generates two separate strands from the hairpin adapter. In some embodiments, cleavage at a cleavage site in the loop region generates a partially double stranded adapter with a double stranded stem and two unpaired strands forming a "Y" structure. Cleavage sites may comprise, for example, uracil and/or deoxyuridine bases, which may be cleaved, for example, using DNA glycosylases, endonucleases, RNAses, and the like and combinations thereof. In some embodiments, the loop of a hairpin adapter contains a uracil residue and can be cleaved using uracil DNA glycosylase and endonuclease VIII. An advantage of using hairpin adapters is that such adapters allow contiguous sequencing of both strands of a double-stranded DNA molecule by covalently attaching one strand to the other. Hairpin adapters also help to minimize the formation of adaptor-dimers during adaptor ligation.

Hairpin adapters are commercially available from New England Biolabs (Ipswich, MA) such as the NEBNext^{®} adaptor, which has sequences that are compatible with Illumina sequencing platforms. The Oxford Nanopore MinION sequencing platform uses a Y-adapter and a hairpin adapter. The Y-adapter is ligated to the one end of a double-stranded DNA molecule, which provides for attachment of the DNA molecule to the sequencing nanopore. The hairpin-like adapter is ligated to the other end of the double-stranded DNA molecule to allow sequencing of both strands of the DNA molecule in series. An exemplary Oxford Nanopore Y adapter comprises a first strand comprising the nucleotide sequence of GGTTGTTTCTGTTGGTGCTGATATTGCGGCGTCTGCTTGGGTGTTTAACCT (SEQ ID NO: 15) and a second strand comprising the nucleotide sequence of GGTTAAACACCCAAGCAGACGCCGCAATATCAGCACCAACAGAAACAACCTTTG AGGCGAGCGGTCAA (SEQ ID NO:16). An exemplary Oxford Nanopore hairpin adapter comprises the nucleotide sequence of CGTTCTGTTTATGTTTCTTGGACACTGATTGACACGGTTTAGTAGAAC (SEQ ID NO: 17)-4(C3 spacer)-28(T)-CAAGAAACATAAACAGAACGT (SEQ ID NO: 18), as described by Karamitros et al. (2015) Nucleic Acids Res. 43(22): e152.

As discussed above, a bead-specific index sequence and a reverse priming site are added to the 3' end of each bead-bound assembled nucleic acid using split-pool enzymatic EDS, wherein the bead-specific index sequence for each bead comprises a different unique identifier sequence. The indexed assembled nucleic acids bound to the beads can be amplified using a forward primer that is complementary to an-adapter sequence and a reverse primer that is complementary to the reverse priming site. For example, if a Y-adapter is used, indexed assembled nucleic acids can be amplified using a forward primer that is complementary to one of the Y-adapter arm sequences and a reverse primer that is complementary to the reverse priming site added by EDS.

### Amplification of Nucleic Acids

Any primer-dependent amplification method may be used for amplification of nucleic acids including, without limitation, polymerase chain reaction (PCR), rolling circle amplification, and isothermal amplification methods such as recombinase polymerase amplification (RPA), loop-mediated isothermal amplification (LAMP), strand displacement amplification (SDA), helicase-dependent amplification (HDA), and nicking enzyme amplification reaction (NEAR), and the like.

In some instances, amplification may be performed by polymerase chain reaction (PCR). PCR primers should be of sufficient length to provide for hybridization to complementary template DNA under annealing conditions. The primers will generally be at least 6 bp in length, including but not limited to e.g., at least 10 bp in length, at least 15 bp in length, at least 16 bp in length, at least 17 bp in length, at least 18 bp in length, at least 19 bp in length, at least 20 bp in length, at least 21 bp in length, at least 22 bp in length, at least 23 bp in length, at least 24 bp in length, at least 25 bp in length, at least 26 bp in length, at least 27 bp in length, at least 28 bp in length, at least 29 bp in length, at least 30 bp in length, and may be as long as 60 bp in length or longer, where the length of the primers will generally range from 18 to 50 bp in length, including but not limited to, e.g., from about 20 to 35 bp in length. In some instances, the template DNA may be contacted with a single primer or a set of two primers (forward and reverse primers), depending on whether primer extension, linear or exponential amplification of the template DNA is desired. Methods of PCR that may be employed in the subject methods include but are not limited to those described in U.S. Pat. Nos.: 4,683,202; 4,683,195; 4,800,159; 4,965,188 and 5,512,462.

In addition to the above components, a PCR reaction mixture may include a polymerase and deoxyribonucleoside triphosphates (dNTPs). The desired polymerase activity may be provided by one or more distinct polymerase enzymes. In many embodiments, the reaction mixture includes at least a Family A polymerase, where representative Family A polymerases of interest include, but are not limited to: *Thermus aquaticus* polymerases, including the naturally occurring polymerase (Taq) and derivatives and homologues thereof, such as Klentaq (as described in Proc. Natl. Acad. Sci USA (1994) 91:2216-2220); *Thermus thermophilus* polymerases, including the naturally occurring polymerase (Tth) and derivatives and homologues thereof, Pol Θ (polymerase theta), as described for example in WO 2019/030149 and WO 2022/086866), and the like. In some embodiments where the amplification reaction is a high fidelity reaction, the reaction mixture may comprise a polymerase enzyme having 3'-5' exonuclease activity, e.g., as may be provided by a Family B polymerase, where Family B polymerases of interest include, but are not limited to: *Thermococcus litoralis* DNA polymerase (Vent) (e.g., as described in Perler et al., Proc. Natl. Acad. Sci. USA (1992) 89:5577*); Pyrococcus* species GB-D (Deep Vent); *Pyrococcus furiosus* DNA polymerase (Pfu) (e.g., as described in Lundberg et al., Gene (1991) 108: 1-6, *Pyrococcus woesei* (Pwo) and the like. Generally, the reaction mixture will include four different types of dNTPs corresponding to the four naturally occurring bases are present, i.e., dATP, dTTP, dCTP and dGTP and in some instances, may include one or more modified nucleotide dNTPs.

Also, a polymerase that preferentially uses dUTP rather than dTTP can be used to perform PCR. Such polymerases include archaeal family B DNA polymerases such as Nanoarchaeum equitans B DNA polymerase, which can use deaminated bases such as uracil and hypoxanthine and performs PCR with higher fidelity than *Thermus aquaticus* (Taq) DNA polymerase (e.g., as described in Choi et al. (2008) Appl. Environ. Microbiol. 74(21): 6563-6569). In addition, engineered polymerases such as Q5U Hot Start High-Fidelity DNA Polymerase from New England Biolabs (Ipswich, MA) and Phusion U DNA polymerase from Thermo Fisher Scientific (Waltham, MA), which contain a mutation in the nucleotide-binding pocket that enables these polymerases to amplify templates containing uracil and inosine bases, may be used to perform PCR with dUTP. The use of polymerases that use UTP is useful for preventing carryover contamination in different PCR runs. The uracil-containing amplicon products of such polymerases can be digested by a uracil-DNA glycosylase to remove residual products from previous PCR amplifications and suppress template contamination between runs. Such methods are described, for example, in PCT Publication No. WO 92/01814.

In addition, one or more PCR additives or enhancing agents may be included to improve the yield of the amplification reaction, for example, by reducing secondary structure in a nucleic acid or mispriming events. Such additives or enhancing agents include, but are not limited to, dimethyl sulfoxide (DMSO), N,N,N-trimethylglycine (betaine), formamide, glycerol, nonionic detergents (e.g., Triton X-100, Tween 20, and Nonidet P-40 (NP-40)), 7-deaza-2'-deoxyguanosine, bovine serum albumin, T4 gene 32 protein, polyethylene glycol, 1,2-propanediol, and tetramethylammonium chloride.

A PCR reaction will generally be carried out by cycling the reaction mixture between appropriate temperatures for annealing, elongation/extension, and denaturation for specific times. Such temperature and times will vary and will depend on the particular components of the reaction including, e.g., the polymerase and the primers as well as the expected length of the resulting PCR product. In some instances, e.g., where nested or two-step PCR are employed the cycling-reaction may be carried out in stages, e.g., cycling according to a first stage having a particular cycling program or using particular temperature(s) and subsequently cycling according to a second stage having a particular cycling program or using particular temperature(s).

Multistep PCR processes may or may not include addition of one or more reagents following the initiation of amplification. For example, in some instances, amplification may be initiated by elongation with the use of a polymerase and, following an initial phase of the reaction, additional reagent(s) (e.g., one or more additional primers, additional enzymes, etc.) may be added to the reaction to facilitate a second phase of the reaction. In some instances, amplification may be initiated with a first primer or a first set of primers and, following an initial phase of the reaction, additional reagent(s) (e.g., one or more additional primers, additional enzymes, etc.) may be added to the reaction to facilitate a second phase of the reaction. In some embodiments, the initial phase of amplification may be referred to as "preamplification".

In particular, the subject methods are applicable to digital PCR techniques. For digital PCR, a sample containing nucleic acids is separated into a large number of partitions before performing PCR. Partitioning can be achieved in a variety of ways, for example, by use of micro well plates, capillaries, emulsions, arrays of miniaturized chambers or nucleic acid binding surfaces. Separation of the sample may involve distributing any suitable portion including up to the entire sample among the partitions. Each partition includes a fluid volume that is isolated from the fluid volumes of other partitions. The partitions may be isolated from one another by a fluid phase, such as a continuous phase of an emulsion, by a solid phase, such as at least one wall of a container, or a combination thereof. In some embodiments, the partitions may comprise droplets disposed in a continuous phase, such that the droplets and the continuous phase collectively form an emulsion.

The partitions may be formed by any suitable procedure, in any suitable manner, and with any suitable properties. For example, the partitions may be formed with a fluid dispenser, such as a pipette, with a droplet generator, by agitation of the sample (e.g., shaking, stirring, sonication, etc.), and the like. Accordingly, the partitions may be formed serially, in parallel, or in batch. The partitions may have any suitable volume or volumes. The partitions may be of substantially uniform volume or may have different volumes. Exemplary partitions having substantially the same volume are monodisperse droplets. Exemplary volumes for the partitions include an average volume of less than about 100, 10 or 1 µL, less than about 100, 10, or 1 nL, or less than about 100, 10, or 1 pL, among others.

After separation of the sample, PCR is carried out in the partitions. The partitions, when formed, may be competent for performance of one or more reactions in the partitions. Alternatively, one or more reagents may be added to the partitions after they are formed to render them competent for reaction. The reagents may be added by any suitable mechanism, such as a fluid dispenser, fusion of droplets, or the like.

In some embodiments, nucleic acids are amplified by emulsion PCR to compartmentalize the amplification reactions of individual DNA molecules. An aqueous PCR mixture with forward and reverse primers is mixed with an oil to create an emulsion. Preferably, each droplet of water in the oil emulsion contains one bead and one molecule of template DNA (e.g., a single assembled nucleic acid of the sequencing library), such that individual molecules are amplified in separate emulsion droplets. After amplification, the emulsion is broken, e.g., using isopropanol and detergent with vortexing. In some embodiments, the gene fragment library and the sequencing library are bound to magnetic beads or superparamagnetic beads prior to amplification, wherein amplification and breaking of the emulsion is followed by magnetic separation of the beads. For a description of emulsion PCR, see, e.g., Kanagal-Shamanna et al. (2016) Methods Mol Biol. 1392:33-42, Zhu et al. (2012) Anal Bioanal Chem. 403(8):2127-43, Zhang et al. (2020) Lab Chip 20(13):2328-2333, Siu et al. (2021) Talanta 221:121593, Zheng et al. (2011) Nat. Protoc. 6(9):1367-1376, and Kojima et al. (2015) Methods Mol. Biol. 2015;1347:87-100.

After PCR amplification, nucleic acids can be quantified by counting the partitions that contain PCR amplicons. Partitioning of the sample allows quantification of the number of different molecules by assuming that the population of molecules follows a Poisson distribution. For a description of digital PCR methods, see, e.g., Hindson et al. (2011) Anal. Chem. 83(22):8604-8610; Pohl and Shih (2004) Expert Rev. Mol. Diagn. 4(1):41-47; Pekin et al. (2011) Lab Chip 11 (13): 2156-2166; Pinheiro et al. (2012) Anal. Chem. 84 (2): 1003-1011; Day et al. (2013) Methods 59(1):101-107.

In some instances, amplification may be carried out under isothermal conditions, e.g., by means of isothermal amplification. Methods of isothermal amplification generally make use of enzymatic means of separating DNA strands to facilitate amplification at constant temperature, such as, e.g., strand-displacing polymerase or a helicase, thus negating the need for thermocycling to denature DNA. Any convenient and appropriate means of isothermal amplification may be employed in the subject methods including but are not limited to: recombinase polymerase amplification (RPA), loop-mediated isothermal amplification (LAMP), strand displacement amplification (SDA), helicase-dependent amplification (HDA), nicking enzyme amplification reaction (NEAR), and the like.

LAMP generally uses a plurality of primers, e.g., 4-6 primers, which may recognize a plurality of distinct regions, e.g., 6-8 distinct regions, of target DNA. Synthesis is generally initiated by a strand-displacing DNA polymerase with two of the primers forming loop structures to facilitate subsequent rounds of amplification. LAMP is rapid and sensitive. In addition, the magnesium pyrophosphate produced during the LAMP amplification reaction may, in some instances be visualized without the use of specialized equipment, e.g., by eye.

RPA combines isothermal recombinase-mediated primer targeting with strand-displacement DNA synthesis (Piepenburg et al. (2006) PLOS Biology. 4 (7): e204). The technique uses two primers together with a recombinase, a single-stranded DNA-binding protein, and a strand-displacing polymerase for amplification. Unlike PCR, heat is not required for melting of the DNA strands. Instead, a recombinase-primer complex is used for localized strand exchange to place oligonucleotide primers at homologous sequences of the DNA template. The single-stranded DNA-binding protein binds to the displaced template strand to prevent the primers from being ejected by branch migration. Dissociation of the recombinase leaves the 3'-end of the primer accessible to the strand displacing DNA polymerase (e.g., the large fragment of *Bacillus subtilis* Pol I), which catalyzes primer extension. Cyclic repetition of this process results in exponential amplification.

SDA generally involves the use of a strand-displacing DNA polymerase (e.g., Bst DNA polymerase, Large (Klenow) Fragment polymerase, Klenow Fragment (3'-5' exo-), and the like) to initiate at nicks created by a strand-limited restriction endonuclease or nicking enzyme at a site contained in a primer. In SDA, the nicking site is generally regenerated with each polymerase displacement step, resulting in exponential amplification.

HDA generally employs: a helicase which unwinds double-stranded DNA unwinding to separate strands; primers, e.g., two primers, that may anneal to the unwound DNA; and a strand-displacing DNA polymerase for extension.

NEAR generally involves a strand-displacing DNA polymerase that initiates elongation at nicks, e.g., created by a nicking enzyme. NEAR is rapid and sensitive, quickly producing many short nucleic acids from a target sequence.

In some instances, entire amplification methods may be combined or aspects of various amplification methods may be recombined to generate a hybrid amplification method. For example, in some instances, aspects of PCR may be used, e.g., to generate the initial template or amplicon or first round or rounds of amplification, and an isothermal amplification method may be subsequently employed for further amplification. In some instances, an isothermal amplification method or aspects of an isothermal amplification method may be employed, followed by PCR for further amplification of the product of the isothermal amplification reaction. In some instances, a sample may be preamplified using a first method of amplification and may be further processed, including e.g., further amplified or analyzed, using a second method of amplification. As a non-limiting example, a sample may be preamplified by PCR and further analyzed by qPCR.

In some instances, the method further comprises monitoring the amplification of a target DNA molecule such as is performed in, e.g., real-time PCR, also referred to herein as quantitative PCR (qPCR).

### DNA Synthesis

Polynucleotides, adapters, and primers can be synthesized by any suitable technique, e.g., solid phase synthesis via phosphoramidite chemistry, as disclosed in U.S. Patent Nos. 4,458,066 and 4,415,732; Adams et al., J. Amer. Chem. Soc. (1983) 105:661-663, Froehler et al., Tetrahedron Lett. (1983) 24:3171-3174, Beaucage et al., Tetrahedron (1992) 48:2223-2311; and Applied Biosystems User Bulletin No. 13 (1 April 1987). Other chemical synthesis methods include, for example, the phosphotriester method described by Narang et al., Meth. Enzymol. (1979) 68:90 and the phosphodiester method disclosed by Brown et al., Meth. Enzymol. (1979) 68:109. Poly(A) or poly(C), or other non-complementary nucleotide extensions may be incorporated into polynucleotides using these same methods. Hexaethylene oxide extensions may be coupled to the polynucleotides by methods known in the art. Cload et al., J. Am. Chem. Soc. (1991) 113:6324-6326; U.S. Patent No. 4,914,210 to Levenson et al.; Durand et al., Nucleic Acids Res. (1990) 18:6353-6359; and Horn et al., Tet. Lett. (1986) 27:4705-4708. Polynucleotides, adapters, and primers can be synthesized in the laboratory, for example, using an automatic synthesizer or by enzymatic DNA synthesis (EDS), as described further below. In some embodiments, polynucleotides having only nucleotides that naturally occur in DNA such as adenine, thymine, guanine and cytosine are synthesized. In other embodiments, unnatural bases, nucleotide analogs, or unnatural base pairs (UBPs) are incorporated into polynucleotides during synthesis. Unnatural nucleobases may have different base pairing and base stacking properties than natural nucleobases. Artificial nucleic acids include peptide nucleic acids (PNAs), morpholino nucleic acids, locked nucleic acid (LNAs), glycol nucleic acids (GNAs), threose nucleic acids (TNAs) and hexitol nucleic acids (HNAs). Modifications may include, but are not limited to, N3- methylation of cytosine, O6-methylation of guanine and N-acetylation of guanine. Exemplary nucleotide analogs include, without limitation, dideoxynucleotides, deaza-nucleotides, aminoallyl nucleotides, thiol containing nucleotides, biotin-containing nucleotides, furan-modified bases, and fluorescent base analogues (e.g., 2-aminopurine, 1,3-diaza-2-oxophenothiazine, 3-MI, 6-MI, 6-MAP, and pyrrolo-dC). Oligonucleotide phosphorothioates (OPS), having an oxygen atom in the phosphate moiety replaced by a sulfur, may also be synthesized. Examples of UBPs include d5SICS and dNaM, which have hydrophobic nucleobases with two fused aromatic rings that form a (d5SICS-dNaM) complex or base pair in DNA. Various modified nucleotides, which can be used in enzymatic synthesis of polynucleotides by terminal deoxynucleotidyl transferase, or variants thereof, without the presence of a template, are described in U.S. Patent Nos. 11,059,849 and 5,763,594. In some cases, modifications to nucleotides may block the polymerization of the nucleotide and/or allow the interaction of the nucleotide with another molecule such as a protein. In some cases, Polynucleotides may be chemically modified after DNA synthesis.

### Enzymatic DNA Synthesis

Enzymatic DNA synthesis (EDS) uses an enzymatic catalyst to carry out the polymerization of nucleotides. EDS can be used to produce primers and other polynucleotides, as well as to add nucleotides to the 3' end of a polynucleotide, for example, for the purpose of adding index sequences, barcodes, priming sequences, restriction sites, or any other desired sequences to polynucleotides or assembled nucleic acids. In some embodiments, EDS is performed with an enzyme that catalyzes the addition of nucleotide-5'-triphosphates to the 3' end of a DNA molecule. More specifically, the process employs, without being limited thereto, enzymes that create a phosphodiester bond between (i) a free 3'-OH group, such as a terminal 3'-OH group of a nucleic acid, and (ii) a 5'-phosphate group of the nucleotide to be added by the enzyme. The polynucleotide to which nucleotides are to be added by EDS may be single- or double-stranded, or may comprise both single- and double stranded portions. When a polynucleotide strand comprises a double-stranded portion, the polynucleotide strand may comprise a 3'-overhang, a recessed 3' end (such that the complementary strand has a 5'-overhang), or a blunt 3'-end (i.e., with no 3' or 5' overhang), to which nucleotides will be added by EDS.

In some embodiments, EDS is performed with an enzyme capable of catalyzing the polymerization of nucleotides without using a template strand. In some embodiments, the enzyme for enzymatic DNA synthesis is a member of the polX family of polymerases, such as DNA polymerase β (Pol β), λ (Pol λ), µ (Pol µ), yeast IV (Pol IV), and terminal deoxyribonucleotidyl transferase (TdT). In some embodiments, the enzyme is a translesion DNA polymerase of type η or ζ, a polynucleotide phosphorylase (PNPase), a template-independent RNA polymerase, a ligase, a template-independent DNA polymerase, a reverse transcriptase, a 9°N DNA polymerase, or terminal deoxynucleotidyl transferases (TdT). Many enzymes that are useful for EDS are expressed by cells of living organisms and can be extracted from these cells or purified from recombinant cultures. These enzymes may have amino acid sequences that are naturally occurring (native to their source organisms) or may comprise one or more modifications relative to their native sequences, such as amino acid insertions, deletions, truncations, substitutions, and/or other modifications.

In some embodiments, an engineered terminal deoxynucleotidyl transferase is used to perform EDS. Various variants of terminal deoxynucleotidyl transferase have been developed for this purpose. See, e.g., PCT Publications No. 2022/0002687. In some embodiments, an engineered reverse transcriptase is used to perform EDS. For example, human immunodeficiency virus type-1 and Moloney murine leukemia virus reverse transcriptases may be used. Engineered Moloney murine leukemia virus reverse transcriptase variants are commercially available such as the SuperScript IV reverse transcriptase from Thermo Fisher (Waltham, MA) and SMARTScribe reverse transcriptase from Clonetech (Mountain View, Calif.).

In some embodiments, an engineered 9°N DNA polymerase is used to perform EDS. Engineered 9°N DNA polymerase variants are commercially available, including duplases from Centrillion Technology Holdings Corporation (Grand Cayman, KY) and the Therminator *Thermococcus sp.* DNA polymerase from New England Biolabs (Ipswich, MA). See also, e.g., Hoff et al. (2020) ACS Synth Biol 9(2):283-293; Gardner et al. (2019) Front. Mol. Biosci. 6:28. Polymerization reactions may be carried out with natural nucleotides or modified nucleotides, or a combination thereof. The nucleotides employed generally consist of a cyclic sugar comprising at least one chemical group at the 5' end and one chemical group at the 3' end and a natural or modified nitrogenous base. The conditions of the reaction medium, in particular the temperature, the pressure, the pH, an optional buffered medium, and other reagents, are chosen in order to make possible optimum functioning of the polymerization enzymes while guaranteeing the integrity of the molecular structures of the different reagents also present.

In some cases, the free end of the nucleotide or nucleotides added to nucleic acids advantageously comprises a protective chemical group in order to prevent multiple addition of nucleotides to the same nucleic acid. The addition of nucleotides protected at their 3' ends with a protective group prevents subsequent polymerization, thus limiting the risk of uncontrolled polymerization. In addition to the prevention of the creation of a phosphodiester bond, a protective group may have other functions, such as to allow interaction with a solid support (e.g., bead) or with other reagents of the reaction medium. For example, a solid support can be covered with molecules, such as proteins, making possible a noncovalent bond between these molecules and the protective chemical group, to allow immobilization on the solid support. Only nucleotides having the protective group would be capable of interacting with the solid support. Nucleic acids which have not had the protective group added can thus be removed based on the lack of interaction between their 3' end and the solid support. If a protection group is used, deprotection is necessary for the satisfactory progression of the synthesis. Deprotection may employ a chemical reaction, an electromagnetic interaction, an enzymatic reaction and/or a chemical or protein interaction.

EDS can be used to add nucleotides to a plurality of nucleic acids simultaneously. EDS reactions can be carried out in parallel for the simultaneous synthesis of a desired sequence on multiple different nucleic acids. Split-pool EDS can be used to add index sequences to assembled nucleic acids while the assembled nucleic acids are immobilized on beads to provide a "bead index" to all clonally amplified molecules immobilized on a bead.

In some embodiments, nucleic acids are immobilized on a solid support that can be washed and exposed to enzymes and buffers via automated liquid handing equipment. For example, the solid support can be a microtiter plate into which reagents are dispensed and removed by a liquid-handling robot. Alternatively, the solid support can be magnetic beads, which can be magnetically separated from a suspension and then resuspended in a new reagent solution in a multi-well plate. Another possible solid support can be an interior surface of a microfluidic device that dispenses reagents to a location in an automated fashion. The immobilization of nucleic acids on a solid support prevents loss of the nucleic acids during wash cycles.

The SYNTAX EDS system is commercially available from DNAScript (South San Francisco, CA). The currently available system provides automated parallel EDS in 96-well plates using an engineered terminal deoxynucleotidyl transferase. For a further description of EDS, see, e.g., U.S. Patent Nos. 10,913,964; 11,268,091; 11,059,849 and U.S. Patent Application Publication No. 2021/0254114.

### Indexing by Split-Pool Synthesis

In some embodiments of the invention, bead-specific index sequences (also known as "barcode" sequences) are added to the clonally amplified assembled nucleic acids bound to beads using a "split and pool" approach. The bead-bound assembled nucleic acids are partitioned into compartments to physically isolate individual beads carrying clonally amplified assembled nucleic acids, such that each bead is separate from every other bead, adding a nucleotide for an index to the bead-bound assembled nucleic acids while they are compartmentalized (where each compartment receives a different nucleotide to add to the 3' ends of the nucleic acid strands on the bead), pooling the bead-bound assembled nucleic acids, and then repeating the process as many times as desired. If the partitioning is random, then the assembled nucleic acids attached to different beads should have received a different set of nucleotides, and therefore a different bead-specific index sequence, by the end of the process. All of the copies of the clonally amplified assembled nucleic acids on the same bead should get the same bead-specific index.

Because the total number of indexing combinations grows exponentially with the number of the split and pool cycles, the split and pool approach can be used to index an almost unlimited number of bead-bound assembled nucleic acids. This becomes more relevant the longer the gene to be synthesized becomes. For example, discovery of perfect copies of a 100 bp gene would require a lower complexity barcode (i.e. shorter in length), than discovery of 10,000 bp gene. One skilled in the art could calculate the probability of recovering a perfect copy based upon the DNA synthesis and gene synthesis methodologies employed and then adjust the number of split and pool cycles in a way to assure a sufficient length of barcode was used to assure experimental outcome success. In all cases, there is no risk to using a barcode longer than one needs, however the risk of randomly synthesizing the same bar code independently on two different beads increases as the length of barcode synthesized decreases (short length random chance of beads having same barcode is higher, drops to zero with increased length) A multi-compartment plate may be used for splitting a sample comprising a plurality of bead-bound assembled nucleic acids. In some embodiments, a device is used to automate the split and pool process, wherein the device is capable of being switched back and forth between: (i) a pooling state in which a sample split between multiple compartments becomes pooled; and (ii) a splitting state in which the sample pooled in (i) becomes split into multiple compartments. The device can be used for repeatedly splitting and pooling a sample comprising a plurality of bead-bound assembled nucleic acids. The device is capable of being readily switched back and forth between a splitting state in the which a pooled sample becomes split into multiple compartments and a pooling state in which a sample that is split between multiple compartments becomes pooled. In the splitting state, the previously pooled sample is split into at least 2, least 4, at least 8, at least 16, at least 48, or at least 96 compartments. In the pooling state, the previously split sample is pooled into a smaller number of compartments. For example, in the pooling state, a sample that is split into n compartments (wherein n = at least 2, least 4, at least 8, at least 16, at least 48, or at least 96, etc.) may be pooled into a smaller number of compartments (e.g., n/2, n/4 or n/8 compartments). In some cases, in the splitting state, the previously pooled sample may be split into at least 8 compartments, whereas in the pooling state the sample (which was previously split between least 8 compartments) may be pooled into a single compartment. The device can be readily switched between the two states, thereby allowing a sample to be split and pooled as many times as needed to provide a unique identifier sequence for each indexed assembled nucleic acid. In some embodiments, the compartments may have a volume in the range of 5 µl to 1 ml, e.g., 10 µl to 500 µl, or 20 µl to 200 µl.

The addition of nucleotides may be catalyzed enzymatically, e.g., by EDS on bead-bound assembled nucleic acids. In particular embodiments, an EDS device may be used for indexing a sample using a split and pool approach, the general goal of which is to add a bead-specific index comprising a unique identifier sequence to each assembled nucleic acid in a sample.

In some embodiments, split-pool EDS is used to add nucleotides one at a time to the 3' ends of the partitioned bead-bound assembled nucleic acids. The bead-bound assembled nucleic acids are partitioned into multiple compartments, a different nucleotide for the bead index is added to each partition, followed by pooling the bead-bound assembled nucleic acids, then repeating the partitioning, addition of a nucleotide, and pooling steps as needed until a sufficient number of nucleotides have been added to provide a unique bead index to the clonally amplified assembled nucleic acids. Typically, no more than 12 to 25 cycles of split-pool EDS will be required to provide a unique bead index for all the beads bound to the clonally amplified assembled nucleic acids produced from assembly of a gene fragment library.

The bead index sequence may be added to assembled nucleic acids, as desired, thereby allowing sequencing data for each assembled nucleic acids to be identified and selective amplification of an indexed assembled nucleic acids with a primer having a sequence complementary to the index sequence. For example, an indexed assembled nucleic acid comprising a perfect copy of a sequence of interest can be selectively amplified out of a pooled mixture using a primer complementary to the index sequence uniquely identifying the bead the assembled nucleic acids is bound to.

A sample may be input into a split-pool device while it is in the splitting state, the pooling state, or in another state (e.g., a "loading" state). Next, the method involves switching the device to the splitting state. In this step, the sample is split into multiple compartments. After splitting, different nucleotides are added to the bead-bound assembled nucleic acids while the sample is split (typically one nucleotide per compartment, wherein the different compartments receive different nucleotides). Next, the method involves switching the device to the pooling state, thereby pooling the compartmentalized sample. The compartmentalization, addition, and pooling steps can then be repeated, as necessary, until the bead-bound assembled nucleic acids are indexed. In these embodiments, the method may comprise repeating the compartmentalization and addition steps multiple times, each repeat followed by a pooling step, except for the final repeat when the pooling step is optional. After these steps have been repeated, the indexed sample may be collected. As would be apparent, suitable mixing and/or washing steps may be performed in between or during any of the steps of the method.

### Sequencing

Any high-throughput technique for sequencing can be used in practicing the invention. For example, DNA sequencing techniques include dideoxy sequencing reactions (Sanger method) using labeled terminators or primers and gel separation in slab or capillary, sequencing by synthesis using reversibly terminated labeled nucleotides, pyrosequencing, 454 sequencing, sequencing by synthesis (e.g., using allele specific hybridization to a library of labeled clones followed by ligation, real time monitoring of the incorporation of labeled nucleotides during a polymerization step), polony sequencing, SOLID sequencing, and the like.

Some high-throughput methods of sequencing comprise a step in which individual molecules are spatially isolated on a solid surface where they are sequenced in parallel. Such solid surfaces may include nonporous surfaces (such as in Solexa sequencing, e.g. Bentley et al, Nature, 456: 53-59 (2008), or Complete Genomics sequencing, e.g. Drmanac et al, Science, 327: 78-81 (2010)), arrays of wells, which may include bead- or particle-bound templates (such as with 454, e.g. Margulies et al, Nature, 437: 376-380 (2005) or Ion Torrent sequencing, e.g., U.S. patent publication 2010/0137143 or 2010/0304982), micromachined membranes (such as with SMRT sequencing of Pacific Biosciences, e.g. Eid et al, Science, 323: 133-138 (2009)), or bead arrays (as with SOLiD sequencing or polony sequencing, e.g. Kim et al, Science, 316: 1481-1414 (2007)). Such methods may comprise amplifying nucleic acids either before or after they are spatially isolated on a solid surface. Amplification may comprise emulsion-based amplification, such as emulsion PCR, or rolling circle amplification.

In some embodiments, sequencing may be performed using the Illumina MiSeq, NextSeq, or HiSeq platforms, which use reversible-terminator sequencing-by-synthesis technologies (see, e.g., Shen et al. (2012) BMC Bioinformatics 13:160; Junemann et al. (2013) Nat. Biotechnol. 31(4):294-296; Glenn (2011) Mol. Ecol. Resour. 11(5):759-769; Thudi et al. (2012) Brief Funct. Genomics 11(1):3-11); the Oxford Nanopore Technologies Inc. MinION, GridION, and PromethION nanopore sequencing platforms, which can be used to determine the sequences of DNA or RNA by monitoring changes in electrical current as nucleic acids are passed through a protein nanopore (see, e.g., Lu et al. (2016) Genomics Proteomics Bioinformatics 14(5):265-279, Petersen et al. (2019) J. Clin. Microbiol. 58(1):e01315-19, Kono et al. (2019) Dev Growth Differ. 61(5):316-326, Deamer et al. (2016) Nat. Biotechnol. 34(5):518-24, Madoui et al. (2015) BMC Genomics 16:327, Szalay et al. (2015) Nat. Biotechnol 33, 1087-1091); the PacBIO Single Molecule, Real-Time (SMRT) sequencing platforms, including the Sequel, HiFi, and RS II sequencing platforms (see, e.g., Ardui et al. (2018) Nucleic Acids Res. 46(5):2159-2168, An et al. (2018) Genes (Basel) 9(1):43), Nakano et al. (2017) Hum Cell. 30(3):149-161), the Omniome sequencing by binding (SBB^{®}) short-read sequencing platform using high fidelity plasmonic nanohole arrays (see, e.g., Cetin et al. (2018) ACS Sens. 3(3):561-568), the Gynapsys compact DNA sequencer, which uses metal oxide semiconductor (CMOS) sequencing chips for electronic data detection and sequencing by synthesis (SBS) chemistry, the Singular Genomics G4 benchtop sequencing platform, which uses SBS chemistry, and the Element Biosciences AVITI^{™} benchtop sequencer, which uses a modified form of SBS chemistry that reduces reagent usage.

These sequencing approaches can thus be used to sequence a sequencing library to identify the unique bead index sequences of clonally amplified assembled nucleic acids comprising perfect copies of a sequence of interest. Short index sequences can also be used in multiplex sequencing of pooled library samples. Accordingly, a correctly assembled nucleic acid comprising a perfect copy of a sequence of interest can then be amplified out of a pooled library sample using a primer that is complementary to the unique bead index sequence of a clonally amplified assembled nucleic acids comprising a perfect copy of the sequence of interest.

In some embodiments, methods of the invention further comprise deep sequencing the amplified indexed assembled nucleic acids on one or more beads. Sequence errors may be introduced at various stages. For example, mistakes may occur during clonal nucleic acid amplification at different cycles of amplification. Errors that occur later will affect fewer numbers of assembled nucleic acids on a bead, wherein as errors that occur earlier during amplification will affect larger numbers of assembled nucleic acids on a bead. Deep sequencing of the assembled nucleic acid products from the beads can resolve these situations and identify perfect copies of the sequence of interest to serve as ideal templates, and identify amplified nucleic acids that contain an unacceptable proportion of non-perfect copies.

### Systems

The present disclosure also describes a system that is useful for practicing the subject methods. The system is used for cell-free identification of a correctly assembled nucleic acid comprising a sequence of interest. In some embodiments, the system may include: a device for performing split-pool EDS, and a processor, wherein the processor is programmed to instruct the device to perform split-pool EDS to add a bead-specific index sequence and to add a reverse priming site to free 3' ends of bead-bound clonally amplified assembled nucleic acids. In some aspects, the system further comprises a sequencer, wherein the sequencer is connected to the processor such that the processor receives sequencing data from the sequencer. In some aspects, the sequencer is inside the device for performing split-pool EDS. In other aspects, the sequencer is a separate device. In some aspects, the system further comprises a memory storage component operably coupled to the processor, wherein the memory storage component is configured to store sequencing data. In some aspects, the processor is further programmed to receive the sequence of the nucleic acid comprising the sequence of interest; design sequences of the plurality of polynucleotides, wherein each polynucleotide comprises a portion of the sequence of interest; and to instruct a synthesizer to synthesize the plurality of polynucleotides.

In some aspects, the system further comprises a chamber for assembly of the plurality of polynucleotides. In some aspects, the chamber further comprises an exonuclease, a DNA polymerase, and a ligase for performing assembly of the plurality of polynucleotides. In some aspects, the chamber comprises reagents for performing Gibson assembly, polymerase chain assembly (PCA), Goldengate assembly, BioBrick assembly, or oligonucleotide ligation. In some aspects, the processor is further programmed to instruct the device to perform the assembly of the plurality of polynucleotides.

In some aspects, the system further comprises a plurality of Y-adapters, a set of polymerase chain reaction (PCR) primers having sequences that are complementary to the sequences of the 5' and 3' arms of the Y-adapters, a PCR primer having a sequence complementary to the reverse priming site; reagents for performing emulsion PCR, beads, or a ligase, or any combination thereof. In some aspects, the beads are non-magnetic beads or magnetic beads (e.g., superparamagnetic beads). In some aspects, the system further comprises reagents for performing Gibson assembly, polymerase chain assembly, Goldengate assembly, BioBrick assembly, or oligonucleotide ligation.

In some aspects, a computer implemented method is used for assembly of a nucleic acid comprising a sequence of interest. The processor may be programmed to perform steps of the computer implemented method comprising: receiving a sequence of a nucleic acid to be assembled from a plurality of polynucleotides; designing sequences of the plurality of polynucleotides, wherein each polynucleotide comprises a portion of the sequence of the nucleic acid comprising the sequence of interest, and the plurality of polynucleotides can be assembled into the full-length sequence of the nucleic acid comprising the sequence of interest; instructing a synthesizer to synthesize the plurality of polynucleotides; instructing a device to add bead-specific index sequences and reverse priming sites to free 3' ends of a plurality of bead-bound clonally amplified assembled nucleic acids produced from the plurality of polynucleotides using split-pool EDS, wherein the bead-specific index sequence for each bead comprises a different unique identifier sequence; receiving sequences of the indexed assembled nucleic acids; analyzing the sequences of the indexed assembled nucleic acids to identify the bead-specific index sequence of a correctly assembled nucleic acid comprising the sequence of interest; and displaying the bead-specific index sequence of the correctly assembled nucleic acid comprising the sequence of interest. In some aspects, the computer implemented method further comprises displaying the sequences of the plurality of polynucleotides. In some aspects, the computer implemented method further comprises displaying sequences of the indexed assembled nucleic acids. In some aspects, the computer implemented method further comprises instructing a device to generate a sequencing library comprising a plurality of assembled nucleic acids produced from assembly of the plurality of polynucleotides.

The methods described herein can be implemented in digital electronic circuitry, or in computer software, firmware, or hardware. The methods herein can be implemented as one or more computer program products, i.e., one or more modules of computer program instructions encoded on a computer readable medium for execution by, or to control the operation of, a data processing apparatus. The computer readable medium can be a machine-readable memory storage device, a machine-readable memory storage substrate, a memory device, a composition of matter effecting a machine-readable propagated signal, or any combination thereof.

A computer program (also known as a program, software, software application, script, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

In a further aspect, the system for performing the computer implemented method, as described, may include a computer containing a processor, a memory storage component, a display component, and other components typically present in general purpose computers. The memory storage component stores information accessible by the processor, including instructions that may be executed by the processor and data that may be retrieved, manipulated or stored by the processor.

The memory storage component may include instructions. For example, the memory storage component includes instructions for analyzing sequencing data of indexed assembled nucleic acids to identify a correctly assembled nucleic acid comprising a perfect copy of the sequence of interest; and identifying the bead-specific index sequence of the correctly assembled nucleic acid comprising the sequence of interest. The computer processor is coupled to the memory storage component and configured to execute the instructions stored in the memory storage component in order to receive a sequence of interest, assemble a nucleic acid comprising the sequence of interest from a plurality of polynucleotides, and identify a correctly assembled nucleic acid comprising the sequence of interest, as described herein. The display component may display information regarding the sequence of interest, sequences of the polynucleotide fragments comprising a portion of the sequence of interest, sequences of the sequenced assembled nucleic acids, and/or the bead-specific index sequence of the correctly assembled nucleic acid comprising the sequence of interest.

The memory storage component may be of any type capable of storing information accessible by the processor, such as a hard-drive, memory card, ROM, RAM, DVD, CD-ROM, USB Flash drive, write-capable, and read-only memories. The processor may be any well-known processor, such as processors from Intel Corporation. Alternatively, the processor may be a dedicated controller such as an ASIC.

The instructions may be any set of instructions to be executed directly (such as machine code) or indirectly (such as scripts) by the processor. In that regard, the terms "instructions," "steps" and "programs" may be used interchangeably herein. The instructions may be stored in object code form for direct processing by the processor, or in any other computer language including scripts or collections of independent source code modules that are interpreted on demand or compiled in advance.

Data may be retrieved, stored or modified by the processor in accordance with the instructions. For instance, although the system is not limited by any particular data structure, the data may be stored in computer registers, in a relational database as a table having a plurality of different fields and records, XML documents, or flat files. The data may also be formatted in any computer-readable format such as, but not limited to, binary values, ASCII or Unicode. Moreover, the data may comprise any information sufficient to identify the relevant information, such as numbers, descriptive text, proprietary codes, pointers, references to data stored in other memories (including other network locations) or information which is used by a function to calculate the relevant data.

In some aspects, the processor and memory storage component may comprise multiple processors and memory storage components that may or may not be stored within the same physical housing. For example, some of the instructions and data may be stored on removable CD-ROM and others within a read-only computer chip. Some or all of the instructions and data may be stored in a location physically remote from, yet still accessible by, the processor. Similarly, the processor may comprise a collection of processors which may or may not operate in parallel.

Components of systems for carrying out the presently disclosed methods are further described in the examples below.

### Kits

Also described is a kit for implementing methods described herein. The kit may include adapters, reagents for amplification of assembled nucleic acids, including primers having sequences that are complementary to primer binding sites of the adapters, reagents for amplification of a sequencing library of assembled nucleic acids, including a primer that is complementary to a primer binding site of an adapter (e.g., one of the arm sequences of a Y-adapter) and a primer that is complementary to an index sequence added to assembled nucleic acids by EDS, nucleotides, and a polymerase (e.g., Taq DNA polymerase). The kit may further comprise reagents for performing emulsion PCR (emPCR) such as an oil for generating emulsions, and reagents for breaking emulsions (e.g., isopropanol, detergent). In some aspects, the kit comprises a plurality of double stranded oligonucleotides/polynucleotides, each oligonucleotide/polynucleotide comprising a portion of a sequence of interest. Different types of kits can be provided according to the requirements of the user. In particular, different oligonucleotides/polynucleotides can be provided in different kits according to the desired sequences to be synthesized. The kit may also comprise reagents for synthesizing the oligonucleotides/polynucleotides of a fragment library by EDS. The kit may further comprise reagents for adding bead index sequences comprising unique identifier sequences and for adding reverse priming sites to assembled nucleic acids using EDS. The EDS reagents may include a reaction medium including an enzyme (e.g., terminal deoxynucleotidyl transferase) for adding nucleotides to the 3' ends of nucleic acids as described herein and natural nucleotides and/or nonnatural nucleotides capable of being used by the enzyme as a substrates. In some aspects, the kit further comprises a device to perform split-pool EDS. The kit may further include reagents for performing nucleic acid assembly such as ligases, restriction enzymes, polymerases, vectors, and other reagents suitable for a selected assembly method such as Gibson assembly, polymerase chain assembly, Goldengate assembly, BioBrick assembly, oligonucleotide ligation, or other assembly method. The kit may further include buffers, washing solutions, a solid support (e.g., magnetic/superparamagnetic beads), and the like. Different types of kits can be provided as a function of an automatic or nonautomatic use. In addition to the above components, the subject kits may further include (in some aspects) instructions for practicing the subject methods using the kit. In some aspects, instructions for cell-free assembly of a nucleic acid comprising a sequence of interest and identification of a correctly assembled nucleic acid comprising the sequence of interest are provided in the kits. These instructions may be present in the subject kits in a variety of forms, one or more of which may be present in the kit. One form in which these instructions may be present is as printed information on a suitable medium or substrate, e.g., on paper on, in the packaging of the kit, in a package insert, and the like. Yet another form of these instructions is a computer readable medium, e.g., diskette, compact disk (CD), DVD, flash drive, SD drive, and the like, on which the information has been recorded. Yet another form of these instructions that may be present is a website address which may be used via the internet to access the information at a removed site.

In some aspects, the kit may include a non-transitory computer-readable medium comprising program instructions that, when executed by a processor in a computer, causes the processor to perform the before-described method for assembly of a nucleic acid comprising a sequence of interest. Said kit may further comprise instructions for assembling a nucleic acid comprising a sequence of interest from a plurality of polynucleotides, and/or a device for performing split-pool enzymatic DNA synthesis (EDS), and/or a plurality of Y-adapters, a set of polymerase chain reaction (PCR) primers having sequences that are complementary to the sequences of the 5' and 3' arms of the Y-adapters, reagents for performing emulsion PCR, beads, a ligase, an exonuclease, a DNA polymerase, or any combination thereof. In some aspects, said beads are magnetic beads, preferably superparamagnetic beads. In further aspects, the kit may further comprise reagents for performing gene assembly.

### Utility

The methods, devices, and systems of the present disclosure find use in assembling one or more nucleic acids comprising a sequence of interest and/or identifying a correctly assembled nucleic acid. The methods are cell-free and allow an assembled nucleic acid comprising a perfect copy of a sequence of interest to be selectively amplified and isolated from a pooled mixture after oligonucleotide assembly. Even rare copies of perfect genes can be easily identified and recovered by the methods disclosed herein. The methods are particularly useful for isolating perfect copies of long genes of interest, which are generally produced at low frequency. Sequence-verified and indexed nucleic acids can be stored in pooled mixtures that allow ready selection and amplification of any individual nucleic acid or subset of nucleic acids on-demand. The disclosed methods can be used for *in vitro* gene synthesis to produce nucleic acids having any desired sequence. For example, nucleic acids comprising an endogenous gene encoding a protein or RNA, an exon, an intron, a wild-type sequence, a major allele, a minor allele, an allele linked to a disease, nucleic acids having mutations (e.g., a deletion, insertion, substitution, single nucleotide polymorphism, frameshift mutation, missense mutation, or nonsense mutation), and engineered nucleic acids having desired functions can be readily assembled and sequence-verified, and a nucleic acid having a perfect copy of the desired sequence can be readily amplified and isolated from pooled mixtures using the disclosed methods. The disclosed methods can be used in conjunction with common gene assembly methods, including, for example, without limitation, Gibson assembly, polymerase chain assembly (PCA), Goldengate assembly, BioBrick assembly, or other oligonucleotide ligation or polymerase-based methods.

### EXAMPLES

The following examples provide further guidance of how to make and use the disclosed subject matter, and are not intended to limit the scope of the invention.

### Example 1

The present disclosure can be used to find perfectly assembled nucleic acid sequences and to amplify them preferentially to obtained pure or purified nucleic acid sequences of interest. A series of steps can be performed by an automated system designed to deliver sequence-perfect nucleic acids without using colony picking or *in vivo* clone propagation. Methods herein do not require propagation of DNA inside a host. Methods herein are cell-free.

### Exemplary Protocol for Assembly of a Nucleic Acid Containing a Gene Sequence

(a) First, a gene fragment library is constructed for assembling a gene sequence of interest. Any method can be used to generate the gene fragment library such as, but not limited to, Gibson Assembly^{®}, polymerase chain assembly (PCA), Goldengate assembly, BioBrick^{®} assembly, or oligo ligation.
(b) Next, Y adapters are ligated to fragments of the library to produce a library of adapter-ligated fragments (also called an "adapted fragment library"). Preferably, to improve ligation efficiency, the fragments of the fragment library are "polished" by end repair and/or adding T and/or A tails before ligating the adapters to the fragments. The Y adapter may be designed to be suitable for emulsion polymerase chain reaction (emPCR), the use of unique identifiers (UIDs), and/or the duplex strand detection technology of Twinstrand Biosciences (Seattle, WA). One or more restriction sites may be included in the adapter-ligated fragments so that the adapters can be cleaved from the correctly assembled nucleic acids using a restriction endonuclease. Sample tracking barcodes on the adapters are not required.
(c) The adapted fragment library is diluted and the adapter-ligated fragments are bound to beads. Preferably, each bead is bound to only one adapter-ligated fragment per bead.
(d) Single molecule emPCR is performed on the adapter-ligated fragments using primers having sequences complementary to the A and B sequences of the Y adapters. The emPCR generates about 10⁵ copies of each fragment on each single bead. The emPCR conditions can be optimized for the length of the fragment library. For example, to amplify 1.5 kb fragments, longer elongation cycles are used than to amplify 3 kb fragments. The emPCR step produces an amplified fragment bead library, wherein each bead is coupled to a single set of clonal copies of a fragment from the original fragment library.
(e) The emulsions are broken, and the beads are purified.
(f) A unique index sequence is added to the fragments on the beads using the split pool enzymatic DNA synthesis. The number of cycles of split pool synthesis depends on the number of beads in the amplified fragment bead library. The number of beads in this indexing step depends on the expected frequency of perfect clonal copies of the assembled sequence of interest. The expected frequency is inversely related to the length of the sequence. Longer assembled sequences require more beads and more cycles of split pool synthesis (because perfect sequences will be less frequent). For example, adding the index sequences to the fragments should require about 12 to 15 cycles, and no more than 25 cycles (4²⁵ index sequences), which is substantially greater than the number of beads that are used (for example, 1×10¹⁶ beads). An excess of index sequences relative to the number of beads helps ensure that each bead will contain a unique index sequence.
(g) A reverse primer priming sequence (primer binding site) is added to the 3'-ends of each indexed assembled nucleic acid on the beads using EDS synthesis. The fixed sequence is typically 20-25 nucleotides in length, although longer or shorter sequence lengths may be used.
(h) The plurality of indexed assembled nucleic acids are amplified using a primer that hybridizes to the Y adapter primer binding site (forward (F) priming sequence) and a primer that hybridizes to the reverse primer priming site. For example, the Y adapter sequence may be amplified with a Pacific Biosciences sequencing primer (Menlo Park, CA), or amplified with an Oxford Nanopore Technologies adapter primer (Oxford, United Kingdom).
(i) The amplified library is sequenced using any suitable next-generation sequencing (NGS) technology. For example, the nanopore sequencing platform of Oxford Nanopore Technologies (Oxford, United Kingdom) or the Single Molecule, Real-Time (SMRT) sequencing platform of Pacific Biosciences (Menlo Park, CA) can be used for sequencing. Sequencing of a small portion of the library is usually enough to identify at least one perfect copy of the nucleic acid sequence of interest. Preferably, the sequencing readouts span the full length of the constructs of the double stranded amplified library, including the (i) forward primer and reverse primer sequences at the 5' termini, and (ii) the forward and reverse primer priming sequences at the 3' termini. The remainder of the amplified library can be stored intact on the indexed beads for later recovery of perfect sequence nucleic acids.
(j) The sequencing results are analyzed to find one or more perfect copies of the assembled nucleic acid sequence of interest. The index sequences identify which beads contain perfect sequence copies of the desired nucleic acid sequence.
(k) Perfect clones are amplified using primers that hybridize to the bead index sequences, in order to selectively amplify only indexed sequences of interest. More specifically, first and second PCR primers are provided to hybridize to and prime from (i) one or more bead specific index sequences that are associated with perfect sequence nucleic acids, and (ii) the immobilized end of the Y adapter that is bound to each bead. The primers can be synthesized by any method, using EDS or phosphoramidite chemistry, for example. In some aspects, amplification is performed on perfect clones bound to their associated beads. In some aspects, amplification is performed on perfect clones that are not bound to beads. For example, amplification can be performed on perfect clones that have been released from their previously associated beads. In some aspects, single-plex PCR amplification can be performed to selectively amplify a single perfect clone using a primer that hybridizes selectively to only one index sequence associated with one clone. In some aspects, a plurality of single-plex PCR amplifications can be performed in separate reaction mixtures to separately amplify a plurality of selected clones, wherein a different primer is used to hybridize to a different index sequence for each different clone that is to be amplified. Alternatively, a plurality of PCR amplifications can be performed simultaneously in a single reaction mixture (multiplex PCR) using a plurality of different primers, wherein each different primer is used to hybridize to a different index sequence associated with each clone that is to be amplified. One benefit of performing single-plex amplifications is that primer competition can be reduced, relative to multiplex PCR, since some index sequences perform better than others, which could lead to differences in representations (biases) in the final amplified products. Also, performing a plurality of single-plex PCR reactions helps increase the likelihood of obtaining a perfect sequence amplification product, if one or more single-plex reactions contain a sequence error that was introduced during PCR amplification.
(l) Perfect clones may be amplified without including Y adapter sequences or index sequences. For example, this can be done using PCR with forward and reverse primers that amplify the sequence of interest, without amplifying the flanking adapter or index sequences. Alternatively, a restriction site can be included in the Y adapters so that the adapters can be cleaved from the adapted construct. Another alternative is to use Gibson assembly into a plasmid, so that the Y adapter sequences are used as a target to recombine the clones into a corresponding recombination site in a plasmid vector. An advantage of this alternative is that *E*. *coli* or another host organism can be used to make large amounts of the perfect clone.

### Additional Advantages

Methods of the present invention are cell-free - no propagation inside cells is required for the workflow. The entire workflow can be automated. The present invention simplifies and enables the identification, selective amplification and isolation of ultra-rare assembled nucleic acids (1 in 1 billion, or 1 in 1 trillion, etc.). The present invention does not have the shortcomings of split and pool strategies that require serial oligo-ligation events and complicated bridging templates (i.e., such as the splint oligo in the method described in O'Huallachain et al. (2020) Commun. Biol. 3(1):213).

## Claims

1. A cell-free method of identifying and amplifying a correctly assembled nucleic acid comprising a sequence of interest, the method comprising:
ligating adapters to the 5' and 3' ends of a plurality of assembled nucleic acids, wherein the adapters comprise an adapter primer binding site and an adapter capture sequence;
binding the plurality of assembled nucleic acids to beads, wherein the beads comprise a capture oligonucleotide attached to the surface of the beads that specifically hybridizes to the adapter capture sequence;
performing clonal amplification of the plurality of assembled nucleic acids bound to the beads using the capture oligonucleotide as a first primer and a second primer that hybridizes to the adapter primer binding site, wherein amplicons produced by clonal amplification of the assembled nucleic acids are bound to the beads, wherein the beads are compartmentalized during clonal amplification such that the amplicons on each bead are separated from other amplicons on other beads so that the amplicons cannot migrate from one bead to another bead;
adding a bead-specific index sequence to free 3' ends of each clonally amplified assembled nucleic acid and amplicons thereof using split-pool enzymatic DNA synthesis (EDS) to produce a plurality of indexed assembled nucleic acids bound to the beads, wherein the bead-specific index sequence for each bead comprises a different unique identifier sequence;
adding a reverse priming site to the free 3' ends of each indexed assembled nucleic acid bound to the beads using EDS;
amplifying the plurality of indexed assembled nucleic acids bound to the beads using a primer that hybridizes to the adapter primer binding site and a primer that hybridizes to the reverse priming site added by EDS;
sequencing at least a portion of the amplified indexed assembled nucleic acids to identify a correctly assembled nucleic acid comprising the sequence of interest;
identifying the bead-specific index sequence of the correctly assembled nucleic acid comprising the sequence of interest; and
performing nucleic acid amplification using a primer that hybridizes to the bead-specific index sequence of the correctly assembled nucleic acid comprising the sequence of interest,
wherein the correctly assembled nucleic acid comprising the sequence of interest is selectively amplified.

2. The method of claim 1, wherein said binding the plurality of assembled nucleic acids to beads is performed under suitable conditions, wherein no more than one assembled nucleic acid binds to each bead, preferably wherein said binding the plurality of assembled nucleic acids to beads is performed with a nucleic acid-to-bead ratio ranging from 0.08 to 0.30.

3. The method of any one of claims 1-2, further comprising deep sequencing the amplified indexed assembled nucleic acids on one or more beads.

4. The method of any one of claims 1-3, further comprising using EDS to add a unique molecular identifier to assembled nucleic acids having different sequences.

5. The method of any one of claims 1-4, wherein the beads are compartmentalized during clonal amplification by isolating each bead in separate emulsion droplets or in separate compartments of a multi-compartment container.

6. The method of any one of claims 1-5, wherein the adapters are linear adapters, Y-adapters, stubby adapters, or hairpin adapters, preferably wherein double-stranded stems of the Y-adapters are ligated to the 5' and 3' ends of the plurality of assembled nucleic acids.

7. The method of any one of claims 1-6, wherein prior to said ligating the adapters, the method further comprises:
assembling a plurality of oligonucleotides into a plurality of polynucleotides, wherein each oligonucleotide comprises a portion of the sequence of interest; and
assembling the plurality of polynucleotides to produce the plurality of assembled nucleic acids.

8. The method of any one of claims 1-7, wherein an isothermal assembly method is used to produce the plurality of assembled nucleic acids, preferably wherein the isothermal method uses an exonuclease, a DNA polymerase, and a ligase.

## Patentansprüche

1. Zellfreies Verfahren zum Identifizieren und Amplifizieren einer korrekt assemblierten Nukleinsäure, die eine interessierende Sequenz umfasst, wobei das Verfahren umfasst:
Ligieren von Adaptern an die 5'- und 3'-Enden einer Vielzahl von assemblierten Nukleinsäuren, wobei die Adapter eine Adapterprimerbindungsstelle und eine Adaptereinfangsequenz umfassen;
Binden der Vielzahl von assemblierten Nukleinsäuren an Kügelchen, wobei die Kügelchen ein an die Oberfläche der Kügelchen gebundenes Einfangoligonukleotid umfassen, das spezifisch an die Adaptereinfangsequenz hybridisiert;
Durchführen klonaler Amplifikation der Vielzahl von an die Kügelchen gebundenen assemblierten Nukleinsäuren unter Verwendung des Einfangoligonukleotids als einen ersten Primer und einen zweiten Primer, der an die
Adapterprimerbindungsstelle hybridisiert, wobei durch klonale Amplifikation der assemblierten Nukleinsäuren produzierte Amplikons an die Kügelchen gebunden werden, wobei die Kügelchen während der klonalen Amplifikation derart kompartimentiert werden, dass die Amplikons auf jedem Kügelchen von anderen Amplikons auf anderen Kügelchen getrennt werden, so dass die Amplikons nicht von einem Kügelchen zu einem anderen Kügelchen migrieren können;
Hinzufügen einer kügelchenspezifischen Indexsequenz zu freien 3'-Enden jeder klonal amplifizierten assemblierten Nukleinsäure und Amplikons davon unter Verwendung enzymatischer Split-Pool-DNA-Synthese (EDS), um eine Vielzahl an die Kügelchen gebundener indexierter assemblierter Nukleinsäuren zu produzieren, wobei die kügelchenspezifische Indexsequenz für jedes Kügelchen eine andere eindeutige Identifikatorsequenz umfasst;
Hinzufügen einer Reverse-Priming-Stelle zu den freien 3'-Enden jeder an die Kügelchen gebundenen indexierten assemblierten Nukleinsäure unter Verwendung von EDS;
Amplifizieren der Vielzahl von an die Kügelchen gebundenen indexierten assemblierten Nukleinsäuren unter Verwendung eines Primers, der an die Adapterprimerbindungsstelle hybridisiert, und eines Primers, der an die durch EDS hinzugefügte Reverse-Priming-Stelle hybridisiert;
Sequenzieren mindestens eines Teils der amplifizierten indexierten assemblierten Nukleinsäuren, um eine korrekt assemblierte Nukleinsäure zu identifizieren, die die interessierende Sequenz umfasst;
Identifizieren der kügelchenspezifischen Indexsequenz der korrekt assemblierten Nukleinsäure, die die interessierende Sequenz umfasst; und
Durchführen einer Nukleinsäureamplifikation unter Verwendung eines Primers, der an die kügelchenspezifische Indexsequenz der korrekt assemblierten Nukleinsäure hybridisiert, die die interessierende Sequenz umfasst, wobei die korrekt assemblierte Nukleinsäure, die die interessierende Sequenz umfasst, selektiv amplifiziert wird.

2. Verfahren nach Anspruch 1, wobei das Binden der Vielzahl von assemblierten Nukleinsäuren an Kügelchen unter geeigneten Bedingungen durchgeführt wird, wobei nicht mehr als eine assemblierte Nukleinsäure an jedes Kügelchen bindet, wobei das Binden der Vielzahl von assemblierten Nukleinsäuren an Kügelchen vorzugsweise mit einem Nukleinsäure-zu-Kügelchen-Verhältnis im Bereich von 0,08 bis 0,30 durchgeführt wird.

3. Verfahren nach einem der Ansprüche 1-2, ferner umfassend Tiefensequenzierung der amplifizierten indexierten assemblierten Nukleinsäuren auf einem oder mehreren Kügelchen.

4. Verfahren nach einem der Ansprüche 1-3, ferner umfassend Verwenden von EDS, um assemblierten Nukleinsäuren mit unterschiedlichen Sequenzen einen eindeutigen molekularen Identifikator hinzuzufügen.

5. Verfahren nach einem der Ansprüche 1-4, wobei die Kügelchen während der klonalen Amplifikation kompartimentiert werden, indem jedes Kügelchen in separaten Emulsionströpfchen oder in separaten Kompartimenten eines Mehrkompartimentbehälters isoliert wird.

6. Verfahren nach einem der Ansprüche 1-5, wobei die Adapter lineare Adapter, Y-Adapter, Stubby-Adapter oder Haarnadeladapter sind, wobei vorzugsweise doppelsträngige Stämme der Y-Adapter an die 5'- und 3'-Enden der Vielzahl von assemblierten Nukleinsäuren ligiert werden.

7. Verfahren nach einem der Ansprüche 1-6, wobei das Verfahren vor dem Ligieren der Adapter ferner Folgendes umfasst:
Assemblieren einer Vielzahl von Oligonukleotiden in eine Vielzahl von Polynukleotiden, wobei jedes Oligonukleotid einen Teil der interessierenden Sequenz umfasst; und Assemblieren der Vielzahl von Polynukleotiden, um die Vielzahl von assemblierten Nukleinsäuren herzustellen.

8. Verfahren nach einem der Ansprüche 1-7, wobei ein isothermes Assemblierungsverfahren verwendet wird, um die Vielzahl von assemblierten Nukleinsäuren herzustellen, wobei das isotherme Verfahren vorzugsweise eine Exonuklease, eine DNA-Polymerase und eine Ligase verwendet.

## Revendications

1. Procédé acellulaire d'identification et d'amplification d'un acide nucléique correctement assemblé comprenant une séquence d'intérêt, le procédé comprenant :
la ligature d'adaptateurs aux extrémités 5' et 3' d'une pluralité d'acides nucléiques assemblés, dans lequel les adaptateurs comprennent un site de liaison d'amorce d'adaptateur et une séquence de capture d'adaptateur ;
la liaison de la pluralité d'acides nucléiques assemblés à des billes, dans lequel les billes comprennent un oligonucléotide de capture fixé à la surface des billes qui s'hybride spécifiquement à la séquence de capture d'adaptateur ;
la réalisation d'une amplification clonale de la pluralité d'acides nucléiques assemblés liés aux billes en utilisant l'oligonucléotide de capture comme première amorce et une seconde amorce qui s'hybride au site de liaison d'amorce d'adaptateur, dans lequel des amplicons produits par amplification clonale des acides nucléiques assemblés sont liés aux billes, dans lequel les billes sont compartimentées pendant l'amplification clonale de sorte que les amplicons sur chaque bille sont séparés des autres amplicons sur d'autres billes, empêchant ainsi les amplicons de migrer d'une bille à une autre ;
l'ajout d'une séquence d'indexation spécifique à une bille pour libérer des extrémités 3' libres de chaque acide nucléique assemblé amplifié de manière clonale et des amplicons de celui-ci à l'aide d'une synthèse enzymatique d'ADN (EDS) fractionnée en pool afin de produire une pluralité d'acides nucléiques assemblés indexés liés aux billes, dans lequel la séquence d'indexation spécifique à une bille pour chaque bille comprend une séquence d'identifiant unique différente ;
l'ajout d'un site d'amorçage inverse aux extrémités libres 3' de chaque acide nucléique assemblé indexé lié aux billes à l'aide d'EDS ;
l'amplification de la pluralité d'acides nucléiques assemblés indexés liés aux billes à l'aide d'une amorce qui s'hybride au site de liaison d'amorce d'adaptateur et d'une amorce qui s'hybride au site d'amorçage inverse ajouté par EDS ;
le séquençage d'au moins une partie des acides nucléiques indexés et amplifiés pour identifier un acide nucléique correctement assemblé comprenant la séquence d'intérêt ;
l'identification de la séquence d'indexation spécifique à une bille de l'acide nucléique correctement assemblé comprenant la séquence d'intérêt ; et
la réalisation d'une amplification d'acide nucléique à l'aide d'une amorce qui s'hybride à la séquence d'indexation spécifique à une bille de l'acide nucléique correctement assemblé comprenant la séquence d'intérêt, dans lequel l'acide nucléique correctement assemblé comprenant la séquence d'intérêt est amplifié de manière sélective.

2. Procédé selon la revendication 1, dans lequel ladite liaison de la pluralité d'acides nucléiques assemblés à des billes est réalisée dans des conditions appropriées, dans lequel pas plus d'un acide nucléique assemblé se lie à chaque bille, de préférence dans lequel ladite liaison de la pluralité d'acides nucléiques assemblés à des billes est réalisée selon un rapport acide nucléique/bille allant de 0,08 à 0,30.

3. Procédé selon l'une quelconque des revendications 1 à 2, comprenant en outre un séquençage profond des acides nucléiques assemblés indexés et amplifiés sur une ou plusieurs billes.

4. Procédé selon l'une quelconque des revendications 1 à 3, comprenant en outre l'utilisation d'une EDS pour ajouter un identifiant moléculaire unique à des acides nucléiques assemblés ayant des séquences différentes.

5. Procédé selon l'une quelconque des revendications 1 à 4, dans lequel les billes sont compartimentées pendant l'amplification clonale en isolant chaque bille dans des gouttelettes d'émulsion distinctes ou dans des compartiments distincts d'un récipient multi-compartiment.

6. Procédé selon l'une quelconque des revendications 1 à 5, dans lequel les adaptateurs sont des adaptateurs linéaires, des adaptateurs en Y, des adaptateurs courts ou des adaptateurs en épingle à cheveux, de préférence dans lequel les tiges double brin des adaptateurs en Y sont ligaturées aux extrémités 5' et 3' de la pluralité d'acides nucléiques assemblés.

7. Procédé selon l'une quelconque des revendications 1 à 6, dans lequel, avant ladite ligature des adaptateurs, le procédé comprend en outre :
l'assemblage d'une pluralité d'oligonucléotides en une pluralité de polynucléotides, dans lequel chaque oligonucléotide comprend une partie de la séquence d'intérêt ; et
l'assemblage de la pluralité de polynucléotides pour produire la pluralité d'acides nucléiques assemblés.

8. Procédé selon l'une quelconque des revendications 1 à 7, dans lequel un procédé d'assemblage isotherme est utilisé pour produire la pluralité d'acides nucléiques assemblés, de préférence dans lequel le procédé isotherme utilise une exonucléase, une ADN polymérase et une ligase.
